(19) Europäisches Patentamt
European Patent Office
Office européen des brevets

(11) EP 2 389 954 A1

(12) **DEMANDE DE BREVET EUROPEEN**

(43) Date de publication:
**30.11.2011 Bulletin 2011/48**

(21) Numéro de dépôt: **11167006.3**

(22) Date de dépôt: **02.11.2006**

(51) Int Cl.:
***A61K 39/395** (2006.01)* ***A61P 7/04** (2006.01)*
***C07K 16/42** (2006.01)*

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **02.11.2005 FR 0511146**

(83) **Déclaration conformément à la règle 32(1) CBE (solution de l'expert)**

(62) Numéro(s) de document de la (des) demande(s) initiale(s) en application de l'article 76 CBE:
**06831055.6 / 1 973 568**

(71) Demandeur: **LFB Biotechnologies**
**91940 Les Ulis (FR)**

(72) Inventeurs:
- **Behrens, Christian**
  **91120, Palaiseau (FR)**
- **Gaucher, Christine**
  **59320, Sequedin (FR)**
- **De Romeuf, Christophe**
  **59130, Lambersart (FR)**

(74) Mandataire: **Hirsch & Associés**
**58, avenue Marceau**
**75008 Paris (FR)**

Remarques:
Cette demande a été déposée le 20-05-2011 comme demande divisionnaire de la demande mentionnée sous le code INID 62.

(54) **Anticorps cytotoxiques dirigés contre des anticorps inhibiteurs du facteur VIII**

(57) La présente invention se rapporte à un anticorps anti-idiotypique dirigé contre un anticorps inhibiteur du facteur VIII humain, ledit anticorps inhibiteur étant dirigé contre la région C2 du facteur VIII humain, dont la région variable de chacune des chaînes légères est codée par une séquence d'acides nucléiques possédant au moins 70% d'identité avec la séquence d'acides nucléiques murine SEQ ID NO : 1, la région variable de chacune de ses chaînes lourdes est codée par une séquence d'acides nucléiques possédant au moins 70% d'identité avec la séquence d'acides nucléiques murine SEQ ID NO : 2, et dont les régions constantes des chaînes légères et des chaînes lourdes sont des régions constantes provenant d'une espèce non-murine, ainsi qu'à l'utilisation de cet anticorps pour activer les récepteurs FcγRIII de cellules immunitaires cytotoxiques, et pour la fabrication d'un médicament, notamment destiné au traitement de l'hémophilie A.

EP 2 389 954 A1

SEQUENCE LISTING

<110> Laboratoire Français du Fractionnement et des Biotechnologies (LFB)

<120> CYTOTOXIC ANTIBODIES DIRECTED AGAINST ANTIBODIES INHIBITING FACTOR VIII

<130> 14C12ch

<160> 32

<170> PatentIn version 3.1

<210> 1

<211> 321

<212> DNA

<213> Mus musculus

<400> 1
gatcttgtgc taactcagtc tccagccacc ctgtctgtga ctccaggaga tagtgtcagt 60

ctttcctgta gggccagcca agatattacc aacacccttc actggtatca tcaaaaatca 120

catgagtctc caaggcttct catcaagtat gtttcccagt ccatctctgg gatcccctcc 180

aggttcagtg gcagtggatc agggacagtt ttcactctca gtatcaacag tgtggagact 240

gaagattttg gagtgtattt ctgtcagcag agtaccagct ggccgtacac attcggaggg 300

gggaccaagt tggaaataaa a 321

<210> 2

<211> 366

<212> DNA

<213> Mus musculus

<400> 2
gaggtccagc ttcagcagtc tggacctgag ctggttaagc ctggggcttc agtgaagctg 60

tcctgcaagg cttctggata cacattcact agctctgtta tgcactggct gaagcagaag 120

tctgggcagg gccttgagtg gattggatat attaatcctt acaatgatgg tactaagtac 180

aatgagaagt tcacagccaa ggccacactg acttcagaca aatcctccag cacagtctac 240

atggagctca gcggcctgac ctctgaggac tttgcggtct attactgtgc acgatcggga 300
ggtttactac gaggttactg gtacttcgat gtctggggcg cagggaccac ggtcaccgtc 360
tcctca 366

<210> 3
<211> 990
<212> DNA
<213> Homo sapiens

<400> 3
gcctccacca agggcccatc ggtcttcccc ctggcaccct cctccaagag cacctctggg 60
ggcacagcgg ccctgggctg cctggtcaag gactacttcc ccgaaccggt gacggtgtcg 120
tggaactcag gcgccctgac cagcggcgtg cacaccttcc cggctgtcct acagtcctca 180
ggactctact ccctcagcag cgtggtgacc gtgccctcca gcagcttggg cacccagacc 240
tacatctgca acgtgaatca caagcccagc aacaccaagg tggacaagaa agttgagccc 300
aaatcttgtg acaaaactca cacatgccca ccgtgcccag cacctgaact cctgggggga 360
ccgtcagtct tcctcttccc cccaaaaccc aaggacaccc tcatgatctc ccggacccct 420
gaggtcacat gcgtggtggt ggacgtgagc cacgaagacc ctgaggtcaa gttcaactgg 480
tacgtggacg gcgtggaggt gcataatgcc aagacaaagc cgcgggagga gcagtacaac 540
agcacgtacc gtgtggtcag cgtcctcacc gtcctgcacc aggactggct gaatggcaag 600
gagtacaagt gcaaggtctc caacaaagcc ctcccagccc ccatcgagaa aaccatctcc 660
aaagccaaag ggcagccccg agaaccacag gtgtacaccc tgcccccatc ccgggatgag 720
ctgaccaaga accaggtcag cctgacctgc ctggtcaaag gcttctatcc cagcgacatc 780
gccgtggagt gggagagcaa tgggcagccg gagaacaact acaagaccac gcctcccgtg 840
ctggactccg acggctcctt cttcctctac agcaagctca ccgtggacaa gagcaggtgg 900
cagcagggga acgtcttctc atgctccgtg atgcatgagg ctctgcacaa ccactacacg 960
cagaagagcc tctccctgtc tccgggtaaa 990

<210> 4
<211> 321
<212> DNA
<213> Homo sapiens

<400> 4
cggactgtgg ctgcaccaag tgtcttcatc ttcccgccat ctgatgagca gttgaaatct 60
ggaactgcct ctgttgtgtg cctgctgaat aacttctatc ccagagaggc caaagtacag 120

tggaaggtgg ataacgccct ccaatcgggt aactcccagg agagtgtcac agagcaggac 180
agcaaggaca gcacctacag cctcagcagc accctgacgc tgagcaaagc agactacgag 240
aaacacaaag tctacgcctg cgaagtcacc catcagggcc tgagctcgcc cgtcacaaag 300
agcttcaaca ggggagagtg t 321

<210> 5
<211> 642
<212> DNA
<213> Artificial Sequence

<220>
<223> Synthetic Construct

<400> 5
gatcttgtgc taactcagtc tccagccacc ctgtctgtga ctccaggaga tagtgtcagt 60
ctttcctgta gggccagcca agatattacc aacacccttc actggtatca tcaaaaatca 120
catgagtctc caaggcttct catcaagtat gtttcccagt ccatctctgg gatcccctcc 180
aggttcagtg gcagtggatc agggacagtt ttcactctca gtatcaacag tgtggagact 240
gaagattttg gagtgtattt ctgtcagcag agtaccagct ggccgtacac attcggaggg 300
gggaccaagt tggaaataaa acggactgtg gctgcaccaa gtgtcttcat cttcccgcca 360
tctgatgagc agttgaaatc tggaactgcc tctgttgtgt gcctgctgaa taacttctat 420
cccagagagg ccaaagtaca gtggaaggtg gataacgccc tccaatcggg taactcccag 480
gagagtgtca cagagcagga cagcaaggac agcacctaca gcctcagcag caccctgacg 540
ctgagcaaag cagactacga gaaacacaaa gtctacgcct gcgaagtcac ccatcagggc 600
ctgagctcgc ccgtcacaaa gagcttcaac aggggagagt gt 642

<210> 6
<211> 1356
<212> DNA
<213> Artificial Sequence

<220>
<223> Synthetic Construct

<400> 6
gaggtccagc ttcagcagtc tggacctgag ctggttaagc ctggggcttc agtgaagctg 60
tcctgcaagg cttctggata cacattcact agctctgtta tgcactggct gaagcagaag 120
tctgggcagg gccttgagtg gattggatat attaatcctt acaatgatgg tactaagtac 180

aatgagaagt tcacagccaa ggccacactg acttcagaca aatcctccag cacagtctac 240

atggagctca gcggcctgac ctctgaggac tttgcggtct attactgtgc acgatcggga 300

ggtttactac gaggttactg gtacttcgat gtctggggcg cagggaccac ggtcaccgtc 360

tcctcagcct ccaccaaggg cccatcggtc ttccccctgg caccctcctc caagagcacc 420

tctgggggca cagcggccct gggctgcctg gtcaaggact acttccccga accggtgacg 480

gtgtcgtgga actcaggcgc cctgaccagc ggcgtgcaca ccttcccggc tgtcctacag 540

tcctcaggac tctactccct cagcagcgtg gtgaccgtgc cctccagcag cttgggcacc 600

cagacctaca tctgcaacgt gaatcacaag cccagcaaca ccaaggtgga caagaaagtt 660

gagcccaaat cttgtgacaa aactcacaca tgcccaccgt gcccagcacc tgaactcctg 720

gggggaccgt cagtcttcct cttcccccca aaacccaagg acaccctcat gatctcccgg 780

acccctgagg tcacatgcgt ggtggtggac gtgagccacg aagaccctga ggtcaagttc 840

aactggtacg tggacggcgt ggaggtgcat aatgccaaga caaagccgcg ggaggagcag 900

tacaacagca cgtaccgtgt ggtcagcgtc ctcaccgtcc tgcaccagga ctggctgaat 960

ggcaaggagt acaagtgcaa ggtctccaac aaagccctcc cagcccccat cgagaaaacc 1020

atctccaaag ccaaagggca gccccgagaa ccacaggtgt acaccctgcc cccatcccgg 1080

gatgagctga ccaagaacca ggtcagcctg acctgcctgg tcaaaggctt ctatcccagc 1140

gacatcgccg tggagtggga gagcaatggg cagccggaga acaactacaa gaccacgcct 1200

cccgtgctgg actccgacgg ctccttcttc ctctacagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaa 1356

<210> 7

<211> 214

<212> PRT

<213> Artificial Sequence

<220>

<223> Synthetic Construct

<400> 7

```
Asp Leu Val Leu Thr Gln Ser Pro Ala Thr Leu Ser Val Thr Pro Gly
1               5                   10                  15

Asp Ser Val Ser Leu Ser Cys Arg Ala Ser Gln Asp Ile Thr Asn Thr
            20                  25                  30

Leu His Trp Tyr His Gln Lys Ser His Glu Ser Pro Arg Leu Leu Ile
        35                  40                  45
```

Lys Tyr Val Ser Gln Ser Ile Ser Gly Ile Pro Ser Arg Phe Ser Gly
    50                  55                  60

Ser Gly Ser Gly Thr Val Phe Thr Leu Ser Ile Asn Ser Val Glu Thr
65                  70                  75                  80

Glu Asp Phe Gly Val Tyr Phe Cys Gln Gln Ser Thr Ser Trp Pro Tyr
                85                  90                  95

Thr Phe Gly Gly Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 8

<211> 452

<212> PRT

<213> Artificial Sequence

<220>

<223> Synthetic Construct

<400> 8

Glu Val Gln Leu Gln Gln Ser Gly Pro Glu Leu Val Lys Pro Gly Ala
1               5                   10                  15

Ser Val Lys Leu Ser Cys Lys Ala Ser Gly Tyr Thr Phe Thr Ser Ser
            20                  25                  30

Val Met His Trp Leu Lys Gln Lys Ser Gly Gln Gly Leu Glu Trp Ile
35 40 45

Gly Tyr Ile Asn Pro Tyr Asn Asp Gly Thr Lys Tyr Asn Glu Lys Phe
50 55 60

Thr Ala Lys Ala Thr Leu Thr Ser Asp Lys Ser Ser Ser Thr Val Tyr
65 70 75 80

Met Glu Leu Ser Gly Leu Thr Ser Glu Asp Phe Ala Val Tyr Tyr Cys
85 90 95

Ala Arg Ser Gly Gly Leu Leu Arg Gly Tyr Trp Tyr Phe Asp Val Trp
100 105 110

Gly Ala Gly Thr Thr Val Thr Val Ser Ser Ala Ser Thr Lys Gly Pro
115 120 125

Ser Val Phe Pro Leu Ala Pro Ser Ser Lys Ser Thr Ser Gly Gly Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr
180 185 190

Val Pro Ser Ser Ser Leu Gly Thr Gln Thr Tyr Ile Cys Asn Val Asn
195 200 205

His Lys Pro Ser Asn Thr Lys Val Asp Lys Lys Val Glu Pro Lys Ser
210 215 220

Cys Asp Lys Thr His Thr Cys Pro Pro Cys Pro Ala Pro Glu Leu Leu
225 230 235 240

Gly Gly Pro Ser Val Phe Leu Phe Pro Pro Lys Pro Lys Asp Thr Leu
245 250 255

Met Ile Ser Arg Thr Pro Glu Val Thr Cys Val Val Val Asp Val Ser
260 265 270

His Glu Asp Pro Glu Val Lys Phe Asn Trp Tyr Val Asp Gly Val Glu
275 280 285

Val His Asn Ala Lys Thr Lys Pro Arg Glu Glu Gln Tyr Asn Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Ala Leu Pro Ala Pro
325 330 335

Ile Glu Lys Thr Ile Ser Lys Ala Lys Gly Gln Pro Arg Glu Pro Gln
340 345 350

Val Tyr Thr Leu Pro Pro Ser Arg Asp Glu Leu Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Val Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Lys Ser Leu Ser Leu
435 440 445

Ser Pro Gly Lys
450

<210> 9

<211> 107

<212> PRT

<213> Mus musculus

<400> 9

Asp Leu Val Leu Thr Gln Ser Pro Ala Thr Leu Ser Val Thr Pro Gly
1 5 10 15

Asp Ser Val Ser Leu Ser Cys Arg Ala Ser Gln Asp Ile Thr Asn Thr
20 25 30

Leu His Trp Tyr His Gln Lys Ser His Glu Ser Pro Arg Leu Leu Ile
35 40 45

Lys Tyr Val Ser Gln Ser Ile Ser Gly Ile Pro Ser Arg Phe Ser Gly
50 55 60

Ser Gly Ser Gly Thr Val Phe Thr Leu Ser Ile Asn Ser Val Glu Thr
65 70 75 80

Glu Asp Phe Gly Val Tyr Phe Cys Gln Gln Ser Thr Ser Trp Pro Tyr
85 90 95

Thr Phe Gly Gly Gly Thr Lys Leu Glu Ile Lys
100 105

<210> 10

<211> 122

<212> PRT

<213> Mus musculus

<400> 10

Glu Val Gln Leu Gln Gln Ser Gly Pro Glu Leu Val Lys Pro Gly Ala
1 5 10 15

Ser Val Lys Leu Ser Cys Lys Ala Ser Gly Tyr Thr Phe Thr Ser Ser
20 25 30

Val Met His Trp Leu Lys Gln Lys Ser Gly Gln Gly Leu Glu Trp Ile
35 40 45

Gly Tyr Ile Asn Pro Tyr Asn Asp Gly Thr Lys Tyr Asn Glu Lys Phe
50 55 60

Thr Ala Lys Ala Thr Leu Thr Ser Asp Lys Ser Ser Ser Thr Val Tyr
65 70 75 80

Met Glu Leu Ser Gly Leu Thr Ser Glu Asp Phe Ala Val Tyr Tyr Cys
85 90 95

Ala Arg Ser Gly Gly Leu Leu Arg Gly Tyr Trp Tyr Phe Asp Val Trp
100 105 110

Gly Ala Gly Thr Thr Val Thr Val Ser Ser
115 120

<210> 11

<211> 330

<212> PRT

<213> Homo sapiens

<400> 11

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270
```

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210> 12

<211> 107

<212> PRT

<213> Homo sapiens

<400> 12

Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105

<210> 13

<211> 11

<212> PRT

<213> Mus musculus

<400> 13

Arg Ala Ser Gln Asp Ile Thr Asn Thr Leu His
1 5 10

<210> 14

<211> 7

<212> PRT

<213> Mus musculus

<400> 14

Tyr Val Ser Gln Ser Ile Ser
1 5

<210> 15

<211> 9

<212> PRT

<213> Mus musculus

<400> 15

Gln Gln Ser Thr Ser Trp Pro Tyr Thr
1 5

<210> 16

<211> 5

<212> PRT

<213> Mus musculus

<400> 16

Ser Ser Val Met His
1 5

<210> 17

<211> 17

<212> PRT

<213> Mus musculus

<400> 17

Tyr Ile Asn Pro Tyr Asn Asp Gly Thr Lys Tyr Asn Glu Lys Phe Thr
1 5 10 15

Ala

<210> 18

<211> 13

<212> PRT

<213> Mus musculus

<400> 18

Ser Gly Gly Leu Leu Arg Gly Tyr Trp Tyr Phe Asp Val
1 5 10

<210> 19

<211> 24

<212> DNA

<213> Mus musculus

<400> 19
actgccatca atcttccact tgac 24

<210> 20

<211> 23

<212> DNA

<213> Mus musculus

<400> 20
ctgagggtgt agaggtcaga ctg 23

<210> 21

<211> 28

<212> DNA

<213> Mus musculus

<400> 21
ttgttcaaga agcacacgac tgaggcac 28

<210> 22

<211> 28

<212> DNA

<213> Mus musculus

<400> 22
gagttccagg tcaaggtcac tggctcag 28

<210> 23

<211> 39

<212> DNA

<213> Artificial Sequence

<220>

<223> Primer

<400> 23
gtatactagt gccgccacca tggttttcac acctcagat 39

<210> 24

<211> 47

<212> DNA

<213> Artificial Sequence

<220>

<223> Primer

<400> 24
tgaagacact tggtgcagcc acagtccgtt ttatttccaa cttggtc 47

<210> 25

<211> 41

<212> DNA

<213> Artificial Sequence

<220>

<223> Primer

<400> 25
ctattactag tgccgccacc atggaatgga gttggatatt t 41

<210> 26

<211> 43

<212> DNA

<213> Artificial Sequence

<220>

<223> Primer

<400> 26
gaccgatggg cccttggtgg aggctgagga gacggtgacc gtg 43

<210> 27

<211> 6

<212> PRT

<213> Mus musculus

<400> 27

Gln Asp Ile Thr Asn Thr
1 5

<210> 28

<211> 3

<212> PRT

<213> Mus musculus

<400> 28

Tyr Val Ser
1

<210> 29

<211> 9

<212> PRT

<213> Mus musculus

<400> 29

Gln Gln Ser Thr Ser Trp Pro Tyr Thr
1 5

<210> 30

<211> 8

<212> PRT

<213> Mus musculus

<400> 30

Gly Tyr Thr Phe Thr Ser Ser Val
1 5

<210> 31

<211> 8

<212> PRT

<213> Mus musculus

<400> 31

Ile Asn Pro Tyr Asn Asp Gly Thr
1 5

<210> 32

<211> 15

<212> PRT

<213> Mus musculus

<400> 32

Ala Arg Ser Gly Gly Leu Leu Arg Gly Tyr Trp Tyr Phe Asp Val
1 5 10 15

## Description

La présente invention se rapporte à un anticorps anti-idiotypique dirigé contre un anticorps inhibiteur du facteur VIII humain, ledit anticorps inhibiteur étant dirigé contre la région C2 du facteur VIII humain, dont la région variable de chacune des chaînes légères est codée par une séquence d'acides nucléiques possédant au moins 70% d'identité avec la séquence d'acides nucléiques murine SEQ ID NO : 1, la région variable de chacune de ses chaînes lourdes est codée par une séquence d'acides nucléiques possédant au moins 70% d'identité avec la séquence d'acides nucléiques murine SEQ ID NO : 2, et dont les régions constantes des chaînes légères et des chaînes lourdes sont des régions constantes provenant d'une espèce non-murine, ainsi qu'à l'utilisation de cet anticorps pour activer les récepteurs FcγRIII de cellules immunitaires cytotoxiques, et pour la fabrication d'un médicament, notamment destiné au traitement de l'hémophilie A.

## Introduction et art antérieur

L'hémophilie A est une affection héréditaire liée à une anomalie du chromosome X, qui se traduit par une incapacité à coaguler chez les personnes atteintes. L'hémophilie A est la plus commune des déficiences affectant la coagulation sanguine : elle touche en France 1 homme sur 5000, ce qui représente 80% des patients atteints d'hémophilie. Cette maladie est le résultat de mutations sur le gène d'une protéine intervenant dans la coagulation, le facteur VIII (FVIII), qui déterminent soit une absence totale de FVIII dans le sang, soit un déficit partiel.

L'autre type d'hémophilie, l'hémophilie B, touche 20% des patients atteints d'hémophilie ; elle est causée par la déficience d'un autre facteur de coagulation, le facteur IX.

Le traitement actuel de l'hémophilie (de type A ou B) consiste à administrer par voie intraveineuse le facteur de coagulation déficient ou manquant. En France, le FVIII destiné au traitement des hémophiles de type A est disponible sous forme de médicaments dérivés du sang fournis par le Laboratoire Français du Fractionnement et des Biotechnologies (LFB) ou par des laboratoires pharmaceutiques internationaux, ainsi que sous forme de médicaments recombinants issus du génie génétique. En effet, l'ADN codant pour le FVIII a été isolé et exprimé dans des cellules de mammifères (Wood et al., Nature (1984) 312 : 330-337), et sa séquence en acides aminés déduite à partir de l'ADNc.

Le FVIII sécrété est une glycoprotéine de masse moléculaire de 300 Kda (2332 acides aminés) jouant un rôle clé dans l'activation de la voie intrinsèque de la coagulation. Le FVIII inactif est constitué de six domaines : A1 (résidus 1-372), A2 (résidus 373-740), B (résidus 741-1648), A3 (résidus 1690-2019), C1 (résidus 2020-2172), et C2 (résidus 2173-2332), de l'extrémité N-terminale à l'extrémité C-terminale. Après sécrétion, le FVIII interagit avec le facteur von Willebrand (vWF) qui le protège des protéases plasmatiques. Le FVIII se dissocie du vWF après clivage par la thrombine. Ce clivage aboutit à l'élimination du domaine B et à la formation d'un hétérodimère. C'est sous cette forme que le FVIII circule dans le plasma. Cet hétérodimère est constitué d'une chaîne lourde (A1, A2) et d'une chaîne légère (A3, C1, C2).

Lorsqu'il est perfusé à un patient hémophile, le FVIII se fixe sur le vWF dans la circulation sanguine du patient. Le FVIII activé agit comme cofacteur du facteur IX activé, accélérant la conversion du facteur X en facteur X activé. Le facteur X activé convertit la prothrombine en thrombine. La thrombine convertit alors le fibrinogène en fibrine et un caillot apparaît.

Le problème majeur rencontré lors de l'administration de FVIII est l'apparition chez le patient d'anticorps dirigés contre le FVIII, appelés « anticorps inhibiteurs ». Ces anticorps neutralisent l'activité procoagulante du FVIII, qui est rendu inactif dés qu'il est perfusé. Ainsi, le facteur de coagulation administré est détruit avant d'avoir pu enrayer l'hémorragie, ce qui constitue une complication grave de l'hémophilie, le traitement devenant inefficace. De plus, certains patients non hémophiles génétiquement peuvent développer des inhibiteurs contre le FVIII endogène : il s'agit d'une hémophilie acquise.

Des études ont montré que la réponse immune anti-FVIII est polyclonale, et principalement dirigée contre les domaines A2 et C2 (Gilles JG et al. (1993) Blood ; 82 : 2452-2461). Un modèle animal a été développé pour étudier la formation d'inhibiteurs du FVIII ; des rats immunisés avec du FVIII humain recombinant montrent une réponse immune rapide, de type polyclonale (Jarvis et al. Thromb Haemost. 1996 Feb; 75 (2) : 318-25).

Les mécanismes par lesquels les anticorps inhibiteurs anti-FVIII interfèrent avec la fonction du FVIII sont nombreux, et incluent l'interférence dans le clivage protéolytique du FVIII et dans l'interaction du FVIII avec différents partenaires comme le vWF, les phospholipides (PL), le facteur IX, le facteur X activé (FXa) ou l'APC (Activated Protein C).

Il existe plusieurs traitements permettant d'atténuer les conséquences de cette réponse immune, comme par exemple les traitements impliquant la desmopressine qui est une hormone synthétique stimulant la production de FVIII, les agents promoteurs de la coagulation comme les concentrés de complexes prothrombiques ou les concentrés de complexes prothrombiques activés, le facteur VIIa recombinant, la plasmaphérèse et les perfusions de quantités importantes ou intermédiaires de FVIII. Toutefois, ces méthodes restent très coûteuses et peu efficaces.

Une autre stratégie, plus récente, envisage l'administration d'anticorps anti-idiotypiques neutralisant les anticorps inhibiteurs (Saint-Rémy JM et al. (1999) Vox Sang ; 77 (suppl 1) : 21-24). Un anticorps monoclonal anti-idiotypique murin, le 14C12, décrit dans le document WO 2004/014955, neutralise in vivo de façon dose-dépendante les propriétés inhibitrices d'un anticorps inhibiteur du FVIII humain. Toutefois, ces anticorps agissent uniquement sur la neutralisation des anticorps. Ils n'ont pas d'action en amont de la sécrétion des anticorps inhibiteurs.

Ainsi, il existe un besoin important de nouveaux outils de traitement permettant à la fois de neutraliser les anticorps inhibiteurs circulant et d'agir en amont de la sécrétion des anticorps inhibiteurs afin de la diminuer.

Le Demandeur a mis au point un nouvel outil utile dans le traitement de l'hémophilie A possédant à la fois une action sur les anticorps inhibiteurs sécrétés et une action en amont sur les précurseurs de plasmocytes sécréteurs d'anticorps inhibiteurs du FVIII, en particulier les cellules B mémoires.

Description

Le Demandeur a mis au point de nouveaux anticorps anti-idiotypiques cytotoxiques, dirigés contre les anticorps inhibiteurs du FVIII, permettant à la fois de neutraliser ces anticorps inhibiteurs circulant en s'y liant, et d'agir sur les cellules B mémoires qui sont à l'origine des plasmocytes qui produisent ces anticorps inhibiteurs, en provoquant leur lyse par un mécanisme d'ADCC (lyse cellulaire dépendante des anticorps).

On entend par anticorps anti-idiotypique un anticorps ayant la capacité d'interagir avec la région variable d'autres anticorps.

L'anticorps cytotoxique anti-idiotypique selon l'invention est dirigé contre tout anticorps inhibiteur se liant à tout domaine du FVIII humain, comme le domaine A1, le domaine A2, le domaine B, le domaine A3 ou encore le domaine C1. Un anticorps anti-idiotypique préféré selon l'invention est un anticorps dirigé contre un anticorps humain inhibiteur se liant au domaine C2 du FVIII.

Le domaine C2 du FVIII contient les sites de liaison pour les phospholipides (PL) et les sites majeurs de liaison au facteur von Willebrand (vWF). La liaison des PL est essentielle pour l'activité physiologique du FVIII, notamment pour la formation du complexe tenase avec le facteur IX (FIX) et le facteur X (FX). Le vWF agit comme une protéine chaperonne, en protégeant le FVIII de la dégradation précoce et de la clairance. Les anticorps inhibiteurs du FVIII dirigés contre le domaine C2 du FVIII sont les inhibiteurs les plus fréquemment rencontrés chez les patients développant des anticorps inhibiteurs. Ainsi, les anticorps anti-idiotypiques préférés selon l'invention ayant pour cible des anticorps inhibiteurs dirigés contre le domaine C2 du FVIII sont particulièrement intéressants, en ce qu'ils sont susceptibles de toucher une majorité de patients atteints d'hémophilie A ayant développé des anticorps inhibiteurs. Ainsi, un objet de l'invention se rapporte à un anticorps monoclonal anti-idiotypique dirigé contre un anticorps inhibiteur du FVIII humain, cet anticorps inhibiteur étant dirigé contre la région C2 du FVIII humain, la région variable de chacune des chaînes légères de l'anticorps monoclonal anti-idiotypique selon l'invention étant codée par une séquence d'acides nucléiques possédant au moins 70% d'identité avec la séquence d'acides nucléiques murine SEQ ID NO : 1, la région variable de chacune de ses chaînes lourdes étant codée par une séquence d'acides nucléiques possédant au moins 70% d'identité avec la séquence d'acides nucléiques murine SEQ ID NO : 2, et les régions constantes de ses chaînes légères et de ses chaînes lourdes étant des régions constantes provenant d'une espèce non-murine. Avantageusement, l'identité des séquences est d'au moins 70%, de manière préférée d'au moins 80%, et de manière encore préférentielle d'au moins 95% ou au moins 99% d'identité. Le pourcentage d'identité est calculé en alignant les 2 séquences à comparer et en comptant le nombre de positions possédant un nucléotide identique, ce nombre étant divisé par le nombre de nucléotides total de la séquence. La dégénérescence du code génétique peut être à l'origine du fait qu'un même acide aminé puisse être codé par plusieurs triplets de nucléotides différents. En tout état de cause, ces différences de séquences n'affectent pas la spécificité de l'anticorps monoclonal pour sa cible, ni sa capacité à neutraliser l'activité inhibitrice des anticorps inhibiteurs cibles en s'y liant. De manière préférentielle, l'affinité de l'anticorps selon l'invention pour sa cible reste identique ou quasi-identique.

Aux fins de l'invention, les expressions équivalentes « anticorps monoclonal » ou « composition d'anticorps monoclonal » se réfèrent à une préparation de molécules d'anticorps possédant une spécificité identique et unique.

L'anticorps monoclonal anti-idiotypique selon l'invention, dont les régions variables des chaînes légères et des chaînes lourdes appartiennent à une espèce différente des régions constantes des chaînes légères et des chaînes lourdes, est qualifié d'anticorps « chimérique ».

Les anticorps anti-idiotypiques chimériques selon l'invention peuvent être construits en utilisant les techniques standard de l'ADN recombinant, bien connues de l'homme du métier, et plus particulièrement en utilisant les techniques de construction des anticorps chimériques décrites par exemple dans Morrison et al., Proc. Natl. Acad. Sci. U.S.A., 81, : 6851-55 (1984), où la technologie de l'ADN recombinant est utilisée pour remplacer la région constante d'une chaîne lourde et/ou la région constante d'une chaîne légère d'un anticorps provenant d'un mammifère non-humain avec les régions correspondantes d'une immunoglobuline humaine. De tels anticorps et leur mode de préparation ont également été décrits dans la publication du brevet EP 173 494, dans le document Neuberger, M.S. et al., Nature 312(5995):604-8 (1985), ainsi que dans le document EP 125 023 par exemple.

Les séquences d'acide nucléique murines SEQ ID NO : 1 et SEQ ID NO : 2 codent respectivement pour le domaine variable de chacune des chaînes légères et le domaine variable de chacune des chaînes lourdes de l'anticorps produit par l'hybridome murin 14C12, disponible depuis le 30 juin 2002 à la Belgian Coordinated Collections of Microorganisms (BCCM), LMBP (plasmid collection, Laboratorium voor Moleculaire Biologie, Universiteit, K.L. Ledeganckstraat 35, 9000 Gent, Belgium), sous le numéro d'accession LMBP 5878CB. Le clone 14C12 a été décrit dans le document WO 2004/014955. Les séquences murines de l'anticorps 14C12 ont été choisies pour coder les régions variables de l'anticorps selon l'invention ou pour dériver des séquences possédant une identité d'au moins 70%, de manière avantageuse au moins 80%, et de manière encore plus avantageuse au moins 90% ou au moins 99%, avec ces séquences en raison des nombreuses caractéristiques avantageuses de la région variable de l'anticorps 14C12 murin, décrites dans le document WO 2004/014955. Un premier aspect avantageux de l'anticorps murin 14C12 est sa capacité à lier un anticorps inhibiteur dirigé contre le domaine C2 du FVIII, l'anticorps BO2C11, qui est un anticorps monoclonal humain spécifique du FVIII dérivé du répertoire naturel d'un patient ayant développé des inhibiteurs (Jacquemin et al. (1998), Blood 92 : 496-506) et à inhiber de manière dose-dépendante la liaison de cet anticorps inhibiteur circulant à sa cible, le domaine C2 du FVIII. Les séquences nucléotidiques et peptidiques des régions variables des chaînes légères et lourdes de l'anticorps BO2C11 sont décrites dans le document WO 01/04269. De plus, l'anticorps murin 14C12 présente la capacité de neutraliser de manière dose-dépendante les propriétés inhibitrices de l'anticorps BO2C11. De plus, il a été montré que l'anticorps 14C12 murin neutralise in vitro 60% de l'inhibition de l'activité du FVIII observée avec des anticorps inhibiteurs du FVIII polyclonaux. L'anticorps murin 14C12 neutralise de plus de manière significative in vitro l'activité inhibitrice du FVIII observée avec des anticorps polyclonaux provenant d'un patient différent du patient à l'origine du clone BO2C11, ce qui indique que l'anticorps murin 14C12 reconnaît un épitope exprimé de manière courante sur les anticorps humains dirigés contre le domaine C2 du FVIII. Concernant les propriétés in vivo de l'anticorps 14C12 murin, il a été montré sur des souris FVIII-/- C57B1 /6 injectées avec du FVIII humain recombinant et l'anticorps inhibiteur BO2C11 que l'anticorps murin 14C12 neutralise de manière dose-dépendante les propriétés inhibitrices de l'anticorps BO2C11, confirmant ainsi l'intérêt de l'anticorps 14C12 murin en thérapie pour traiter les patients atteints d'hémophilie A ayant développé des inhibiteurs contre le domaine C2 du FVIII. Par ailleurs, l'anticorps murin 14C12 se lie avec une haute affinité à l'anticorps BO2C11, avec des valeurs de $k_{on}$ et $k_{off}$ de $10^5$ $M^{-1}$ $s^{-1}$ et $10^{-5}$ $s^{-1}$ respectivement. De plus, il a été démontré que la partie variable de la chaîne lourde de l'anticorps murin 14C12 contient à la fois l'image interne du domaine C2 faite de 13 acides aminés identiques ou homologues, et plusieurs résidus de contact pour la partie variable de BO2C11. Enfin, il a été démontré que l'anticorps murin 14C12 n'inhibe pas la liaison du FVIII au vWF ou aux PL. Ainsi, l'administration de l'anticorps murin 14C12 à des patients atteints d'hémophilie A ayant développé des anticorps inhibiteurs dirigés contre le domaine C2 du FVIII ne provoque pas une inhibition indésirable des propriétés fonctionnelles du FVIII. Avantageusement, l'anticorps monoclonal anti-idiotypique selon l'invention possède toutes les propriétés avantageuses liées à la région variable de l'anticorps 14C12.

L'anticorps selon l'invention possède en outre des régions constantes de ses chaînes légères et lourdes appartenant à une espèce non-murine. A cet égard, toutes les familles et espèces de mammifères non-murins sont susceptibles d'être utilisées, et en particulier l'homme, le singe, les muridés (sauf la souris), les suidés, les bovidés, les équidés, les félidés, les canidés, par exemple, ainsi que les oiseaux, cette liste n'étant pas exhaustive.

De manière préférentielle, la région variable de chacune des chaînes légères de l'anticorps anti-idiotypique de l'invention est codée par la séquence d'acide nucléique murine SEQ ID NO : 1, et la région variable de chacune de ses chaînes lourdes est codée par la séquence d'acide nucléique murine SEQ ID NO : 2, les régions constantes de ses chaînes légères et de ses chaînes lourdes étant des régions constantes provenant d'une espèce non-murine.

De manière avantageuse, l'anticorps monoclonal anti-idiotypique selon l'invention est produit par une cellule sous forme de composition d'anticorps, dont le ratio taux de fucose/taux de galactose des structures glycanniques présentes sur le site de glycosylation de la région Fc des anticorps, est inférieur ou égal à 0,6. Par « site de glycosylation de la région Fc », on entend désigner l'asparagine 297 (Asn 297, numérotation de Kabat), qui porte un site de N-glycosylation. En effet, la région constante Fc des anticorps est constituée de 2 domaines globulaires nommés $CH_2$ et $CH_3$. Les 2 chaînes lourdes interagissent étroitement au niveau des domaines $CH_3$ tandis qu'au niveau des domaines $CH_2$, la présence, sur chacune des 2 chaînes, d'un N-glycanne biantenné, lié à l'Asn 297, contribue à un écartement des 2 domaines. Ainsi, dans la mise en oeuvre de l'invention, les structures glycanniques portées par la totalité des sites de glycosylation présents dans la composition d'anticorps montrent un ratio taux de fucose/taux de galactose inférieur ou égal à 0,6, pour lequel il a été démontré dans la demande de brevet FR 03 12229 qu'il est optimal pour conférer une forte activité ADCC (« cytotoxicité cellulaire dépendante des anticorps » ) aux anticorps. Les anticorps anti-idiotypiques selon l'invention ont donc pour particularité et avantage de présenter une capacité accrue d'activer par leur région Fc les récepteurs Fc des cellules effectrices cytotoxiques, et en particulier le récepteur FcγRIIIA (appelé aussi CD16), récepteur activateur des cellules effectrices cytotoxiques. Les cellules effectrices pouvant être activées par l'anticorps monoclonal anti-idiotypique selon l'invention sont par exemple des cellules NK (Natural Killer), des macrophages, des neutrophiles, les lymphocytes CD8, les lymphocytes Tγδ, les cellules NKT, les éosinophiles, les basophiles ou les mastocytes. De manière préférée, l'anticorps anti-idiotypique selon l'invention permet de recruter des cellules NK. L'anticorps monoclonal anti-idiotypique selon l'invention est dit cytotoxique, et va permettre de recruter via sa région Fc des cellules effectrices, qui vont détruire la cellule portant à sa surface la cible de l'anticorps anti-idiotypique selon l'invention.

Ainsi, l'anticorps selon l'invention permet la destruction des cellules exprimant à leur surface ledit anticorps inhibiteur du FVIII, qui sont les précurseurs de plasmocytes sécréteurs d'anticorps inhibiteurs de FVIII, en particulier les cellules B mémoires.

De manière préférée, les cellules cibles sont les cellules B mémoires.

Ainsi, l'anticorps monoclonal anti-idiotypique selon l'invention va d'une part inhiber la fixation de l'anticorps inhibiteur au FVIII en se liant à l'idiotope de l'anticorps inhibiteur, et d'autre part provoquer la lyse des cellules B mémoires exprimant à leur surface l'anticorps inhibiteur cible et précurseurs des plasmocytes sécrétant les anticorps inhibiteurs, ces deux effets participant à la réduction de l'effet inhibiteur des anticorps inhibiteurs du FVIII produits par le patient hémophile.

De manière préférée, l'anticorps inhibiteur anti-FVIII contre lequel l'anticorps anti-idiotypique de l'invention est dirigé est l'anticorps BO2C11. Cet anticorps est un anticorps monoclonal IgG4kappa humain spécifique du FVIII dérivé du répertoire naturel d'un patient atteint d'hémophilie A présentant des inhibiteurs (Jacquemin MG et al. (1998) Blood 92 : 496-506) . L'anticorps BO2C11 reconnaît le domaine C2 et inhibe la liaison du FVIII au vWF et aux phospholipides (PL), ce mécanisme d'action étant le plus communément rencontré chez les patients présentant des anticorps inhibiteurs spécifiques du domaine C2 du FVIII. De plus, le site de liaison exact de l'anticorps BO2C11 sur le domaine C2 a été identifié par analyse aux rayons X de cristaux de fragments Fab et du domaine C2 (Spiegel PC et al. (2001) Blood 98 : 13-19). Les séquences en acides aminés et en nucléotides des régions variables des chaînes lourdes et des chaînes légères de l'anticorps BO2C11 ont été décrites dans le document WO 01/04269, datant de 2000.

Ainsi, l'anticorps anti-idiotypique selon l'invention reconnaît l'anticorps BO2C11 circulant, ainsi que l'anticorps BO2C11 membranaire (BCR) situé à la surface du clone de cellule B mémoire BO2C11. L'anticorps anti-idiotypique selon l'invention va donc d'une part neutraliser l'anticorps circulant en s'y fixant, et d'autre part se lier à l'immunoglobuline membranaire, ce qui va être suivi par la liaison entre la région Fc de l'anticorps selon l'invention et les cellules cytotoxiques par l'intermédiaire du récepteur Fc de ces cellules cytotoxiques. L'anticorps selon l'invention va donc participer à la lyse des cellules B mémoires exprimant l'inmmunoglobuline BO2C11 à leur surface.

De manière préférée, les régions constantes de chacune des chaînes légères et de chacune des chaînes lourdes de l'anticorps anti-idiotypique selon l'invention sont des régions constantes humaines. Ce mode de réalisation préféré de l'invention permet de diminuer l'immunogénicité de l'anticorps chez l'homme et par là même d'améliorer son efficacité lors de son administration thérapeutique chez l'homme.

Dans un mode de réalisation préféré de l'invention, la région constante de chacune des chaînes légères de l'anticorps selon l'invention est de type κ (kappa). Tout allotype convient à la réalisation de l'invention, par exemple Km (1), Km(1, 2), Km(1, 2, 3) ou Km(3) mais l'allotype préféré est Km(3).

Dans un autre mode de réalisation complémentaire, la région constante de chacune des chaînes légères de l'anticorps selon l'invention est de type λ (lambda). Dans un aspect particulier de l'invention, et notamment lorsque les régions constantes de chacune des chaînes légères et de chacune des chaînes lourdes de l'anticorps selon l'invention sont des régions humaines, la région constante de chacune des chaînes lourdes de l'anticorps est de type γ (gamma). Selon cette variante, la région constante de chacune des chaînes lourdes de l'anticorps peut être de type γ1, γ2, ou γ3, ces trois types de régions constantes présentant la particularité de fixer le complément humain, ou encore de type γ4. Les anticorps possédant une région constante de chacune des chaînes lourdes de type γ appartiennent à la classe des IgG. Les immunoglobulines de type G (IgG), sont des hétérodimères constitués de 2 chaînes lourdes et de 2 chaînes légères, liées entre elles par des ponts disulfures. Chaque chaîne est constituée, en position N-terminale, d'une région ou domaine variable (codée par les gènes réarrangés V-J pour la chaîne légère et V-D-J pour la chaîne lourde) spécifique de l'antigène contre lequel l'anticorps est dirigé, et en position C-terminale, d'une région constante, constituée d'un seul domaine CL pour la chaîne légère ou de 3 domaines (CH1, CH2 et CH3) pour la chaîne lourde. L'association des domaines variables et des domaines $CH_1$ et CL des chaînes lourdes et légères forme les régions Fab, qui sont connectées à la région Fc par une région charnière très flexible permettant à chaque Fab de se fixer à sa cible antigénique tandis que la région Fc, médiatrice des propriétés effectrices de l'anticorps, reste accessible aux molécules effectrices telles que les récepteurs FcγR et le C1q. La région Fc, constituée des 2 domaines globulaires $CH_2$ et $CH_3$, est glycosylée au niveau du domaine $CH_2$ avec la présence, sur chacune des 2 chaînes, d'un N-glycanne biantenné, lié à l'Asn 297.

De manière préférée, la région constante de chacune des chaînes lourdes de l'anticorps est de type γ1, car un tel anticorps montre une capacité à engendrer une activité ADCC chez le plus grand nombre d'individus (humains). A cet égard, tout allotype convient à la réalisation de l'invention, par exemple G1m (3), G1m(1,2,17), G1m(1,17) ou G1m (1,3). De manière préférentielle, l'allotype est G1m(1,17).

Dans un aspect particulier de l'invention, la région constante de chacune des chaînes lourdes de l'anticorps est de type γ1, et elle est codée par une séquence d'acides nucléiques possédant au moins 70% d'identité avec la séquence d'acide nucléique humaine SEQ ID NO : 3, la région constante de chacune de ses chaînes légères étant codée par une séquence possédant au moins 70% d'identité avec la séquence d'acide nucléique humaine SEQ ID NO : 4.

De manière avantageuse, l'identité des séquences citées ci-dessus est d'au moins 80%, et de manière particulièrement avantageuse d'au moins 90% ou 99%, les modifications de séquences ne modifiant pas les propriétés fonctionnelles de l'anticorps selon l'invention.

De manière préférée, la région constante de chacune des chaînes lourdes de l'anticorps selon l'invention est de type γ1 et est codée par la séquence d'acides nucléiques humaine SEQ ID NO : 3 et la région constante de chacune de ses chaînes légères est codée par la séquence d'acides nucléiques humaine SEQ ID NO : 4.

Ainsi, un tel anticorps possède une région variable murine et une région constante humaine, avec des chaînes lourdes de type γ1. Cet anticorps appartient donc à la sous-classe des IgG1 humaines. Cet anticorps possède deux chaînes légères dont le domaine variable est codé par la séquence d'acide nucléique murine SEQ ID NO : 1 et la région constante humaine est codée par la séquence d'acide nucléique SEQ ID NO : 4, et deux chaînes lourdes dont le domaine variable est codé par la séquence d'acide nucléique murine SEQ ID NO : 2 et la région constante est codée par la séquence d'acide nucléique humaine SEQ ID NO : 3.

Dans un autre aspect de l'invention, chacune des chaînes légères de l'anticorps selon l'invention est codée par une séquence possédant au moins 70% d'identité avec la séquence d'acide nucléique chimérique murine-humaine SEQ ID NO : 5, et chacune des chaînes lourdes est codée par une séquence possédant au moins 70% d'identité avec la séquence d'acide nucléique chimérique murine-humaine SEQ ID NO : 6. De manière particulièrement avantageuse, les identités de séquences sont d'au moins 80%, et de manière encore plus avantageuse d'au moins 90% ou au moins 99%, les modifications de séquence ne modifiant pas la spécificité de l'anticorps ni ses propriétés fonctionnelles.

De manière préférentielle, chacune des chaînes légères de l'anticorps selon l'invention est codée par la séquence d'acide nucléique chimérique murine-humaine SEQ ID NO : 5, et chacune des chaînes lourdes est codée par la séquence d'acide nucléique chimérique murine-humaine SEQ ID NO : 6.

La séquence d'acide nucléique chimérique murine-humaine SEQ ID NO : 5 codant pour chacune des chaînes légères de l'anticorps est obtenue par fusion de la séquence d'acide nucléique murine SEQ ID NO : 1 codant pour le domaine variable de chacune des chaînes légères de l'anticorps et de la séquence d'acide nucléique humaine SEQ ID NO : 4 codant pour la région constante de chacune des chaînes légères de l'anticorps. La séquence d'acide nucléique chimérique murine-humaine SEQ ID NO : 6 codant pour chacune des chaînes lourdes de l'anticorps est obtenue par fusion de la séquence d'acide nucléique murine SEQ ID NO : 2 codant pour le domaine variable de chacune des chaînes lourdes de l'anticorps et de la séquence d'acide nucléique humaine SEQ ID NO : 3 codant pour la région constante de chacune des chaînes lourdes de l'anticorps. De manière avantageuse, chacune des chaînes légères de l'anticorps selon l'invention possède une séquence peptidique ayant au moins 70% d'identité avec la séquence peptidique SEQ ID NO : 7, et chacune des chaînes lourdes de l'anticorps selon l'invention possède une séquence peptidique ayant au moins 70% d'identité avec la séquence peptidique SEQ ID NO : 8. De manière particulièrement avantageuse, l'identité entre les séquences est d'au moins 80%, 90%, ou encore 95%, ou 99%, les modifications de séquence ne modifiant pas la spécificité de l'anticorps ni ses propriétés fonctionnelles.

De manière préférée, la séquence peptidique de chacune des chaînes légères de l'anticorps selon l'invention est la séquence peptidique SEQ ID NO : 7, et la séquence peptidique de chacune des chaînes lourdes de l'anticorps selon l'invention est la séquence peptidique SEQ ID NO : 8.

Ainsi, lorsque chacune des chaînes légères de l'anticorps est codée par la séquence d'acide nucléique chimérique murine-humaine SEQ ID NO : 5, et que chacune des chaînes lourdes est codée par la séquence d'acide nucléique chimérique murine-humaine SEQ ID NO : 6, la séquence peptidique de chacune des chaînes légères, déduite de la séquence d'acide nucléique SEQ ID NO : 5 est la séquence SEQ ID NO : 7 et la séquence peptidique de chacune des chaînes lourdes, déduite de la séquence d'acide nucléique SEQ ID NO : 6, est la séquence SEQ ID NO : 8. L'anticorps selon l'invention peut être produit par toute lignée cellulaire, et plus particulièrement par une lignée cellulaire produisant des anticorps ayant une forte affinité pour le CD16.

De manière particulièrement avantageuse, l'anticorps de l'invention est produit par une lignée cellulaire de myélome de rat. La lignée productrice de l'anticorps selon l'invention est une caractéristique importante puisqu'elle confère à l'anticorps certaines de ses propriétés particulières. En effet, le moyen d'expression des anticorps est à l'origine des modifications post-traductionnelles, notamment des modifications de la glycosylation, qui peuvent varier d'une lignée cellulaire à l'autre, et ainsi conférer des propriétés fonctionnelles différentes à des anticorps ayant pourtant des structures primaires identiques.

Dans un mode de réalisation préféré, l'anticorps est produit dans le myélome de rat YB2/0 (cellule YB2/3HL.P2.G11.16Ag.20, déposée à l'American Type Culture Collection sous le numéro ATCC CRL-1662). Cette lignée a été choisie en raison de sa capacité à produire des anticorps présentant une activité ADCC améliorée par rapport à des anticorps de même structure primaire produits par exemple dans CHO, et de l'absence de production d'immunoglobulines endogènes. Ainsi, les anticorps selon l'invention produits dans la lignée cellulaire YB2/0 présentent une capacité accrue d'activer par leur région Fc les récepteurs Fc des cellules cytotoxiques par rapport à un anticorps de même structure primaire produit dans une autre lignée cellulaire. De plus, cette lignée cellulaire a pour particularité et avantage de produire des anticorps sous forme de composition d'anticorps, dont le ratio taux de fucose/taux de galactose des structures glycanniques présentes sur le site de glycosylation de la région Fc des anticorps, est inférieur ou égal à 0,6.

De manière avantageuse, l'anticorps selon l'invention est susceptible d'être produit par le clone R565, déposé le 25 octobre 2005, sous le numéro I-3510 à la Collection Nationale de Culture des Microorganismes (CNCM, 25 rue du Docteur Roux, 75724 Paris cedex 15).

Avantageusement, l'anticorps selon l'invention est l'anticorps EMAB565, produit par le clone R565. Chacune des chaînes légères de l'anticorps EMAB565 est codée par la séquence d'acide nucléique chimérique murine-humaine SEQ ID NO : 5, et chacune de ses chaînes lourdes est codée par la séquence d'acide nucléique chimérique murine-humaine SEQ ID NO : 6. Cet anticorps chimérique entre en compétition avec l'anticorps murin 14C12 pour la fixation du FVIII et possède une activité cytotoxique accrue, très supérieure à celle du 14C12 murin, qui peut être attribuée pour partie à la glycosylation particulière du N-glycanne de la chaîne lourde de ces anticorps. En effet, le clone R565 a pour particularité de produire une composition d'anticorps EMAB565 possédant un ratio taux de fucose/taux de galactose inférieur à 0,6, pour lequel il a été démontré dans la demande de brevet FR 03 12229 qu'il est optimal pour conférer une forte activité ADCC aux anticorps. Cet anticorps est donc particulièrement intéressant comme outil thérapeutique pour le traitement de pathologies dont les cellules à cibler expriment des anticorps inhibiteurs du FVIII. L'invention s'entend aussi de tout anticorps monoclonal possédant substantiellement les mêmes caractéristiques que l'anticorps EMAB565.

Un autre objet de l'invention se rapporte à un vecteur d'expression de la chaîne légère d'un anticorps selon l'invention. Ce vecteur est le vecteur permettant l'expression d'un anticorps selon l'invention dont la chaîne légère est codée par la séquence d'acide nucléique SED ID NO : 5, dont la séquence peptidique déduite est la séquence SEQ ID NO : 7. Ce vecteur est une molécule d'acide nucléique dans laquelle la séquence d'acide nucléique murine SEQ ID NO : 1 codant pour le domaine variable de chacune des chaînes légères de l'anticorps et la séquence d'acide nucléique SEQ ID NO : 4 codant pour la région constante de chacune des chaînes légères de l'anticorps ont été insérées, afin de les introduire et de les maintenir dans une cellule hôte. Il permet l'expression de ces fragments d'acide nucléique étrangers dans la cellule hôte car il possède des séquences indispensables (promoteur, séquence de polyadénylation, gène de sélection) à la sélection et à l'expression. De tels vecteurs sont bien connus de l'homme du métier, et peuvent être un adénovirus, un rétrovirus, un plasmide ou un bactériophage, cette liste n'étant pas limitative. De plus, toute cellule de mammifère peut être utilisée comme cellule hôte, c'est-à-dire comme cellule exprimant l'anticorps selon l'invention, par exemple YB2/0, CHO, CHO dhfr- (par exemple CHO DX B11, CHO DG44), CHO Lec13, SP2/0, NS0, 293, BHK ou COS.

Un autre objet de l'invention se rapporte à un vecteur d'expression de la chaîne lourde d'un anticorps selon l'invention. Ce vecteur est le vecteur permettant l'expression d'un anticorps selon l'invention dont la chaîne lourde est codée par la séquence d'acide nucléique SED ID NO : 6, dont la séquence peptidique déduite est la séquence SEQ ID NO : 8. Ce vecteur est une molécule d'acide nucléique dans laquelle la séquence d'acide nucléique murine SEQ ID NO : 2 codant pour le domaine variable de chacune des chaînes lourdes de l'anticorps et la séquence d'acide nucléique humaine SEQ ID NO : 3 codant pour la région constante de chacune des chaînes lourdes de l'anticorps ont été insérées, afin de les introduire et de les maintenir dans une cellule hôte. Il permet l'expression de ces fragments d'acide nucléique étrangers dans la cellule hôte car il possède des séquences indispensables (promoteur, séquence de polyadénylation, gène de sélection) à cette expression. Tout comme indiqué précédemment, le vecteur peut être par exemple un plasmide, un adénovirus, un rétrovirus ou un bactériophage, et la cellule hôte peut être toute cellule de mammifère, par exemple YB2/0, CHO, CHO dhfr- (CHO DX B11, CHO DG44), CHO Lec13, SP2/0, NS0, 293, BHK ou COS. Un anticorps produit par co-expression des vecteurs d'expression de la chaîne lourde et de la chaîne légère dans la cellule YB2/0 est illustré par l'anticorps EMAB565, produit par le clone R565 (déposé sous le numéro d'enregistrement I-3510 à la CNCM). Cet anticorps induit une cytotoxicité très supérieure à celle induite par l'anticorps 14C12 murin en présence de cellules NK humaines. De plus, l'anticorps EMAB565 induit une sécrétion d'IL-2 (interleukine 2) par les cellules Jurkat-CD16 beaucoup plus forte que l'anticorps 14C12 murin. L'anticorps EMAB565, pouvant être produit par culture du clone R565 dans un milieu de culture et à des conditions permettant l'expression des vecteurs précédemment décrits, est donc un outil des plus intéressants susceptible de faire progresser la thérapie et le diagnostic des pathologies impliquant le BO2C11, et plus particulièrement l'hémophilie A, ainsi que la recherche dans ce domaine.

Un autre objet particulier de l'invention est une lignée cellulaire stable exprimant un anticorps selon l'invention.

De manière avantageuse, la lignée cellulaire stable exprimant un anticorps selon l'invention est choisie parmi le groupe consistant en : SP2/0, YB2/0, IR983F, un myélome humain comme Namalwa ou toute autre cellule d'origine humaine comme PER.C6, les lignées CHO, notamment CHO-K-1, CHO-Lec10, CHO-Lec1, CHO-Lec13, CHO Pro-5, CHO dhfr- (CHO DX B11, CHO DG44), ou d'autres lignées choisies parmi Wil-2, Jurkat, Vero, Molt-4, COS-7, 293-HEK, BHK, K6H6, NS0, SP2/0-Ag 14 et P3X63Ag8.653. De manière préférée, la lignée utilisée est le myélome de rat YB2/0. Cette lignée a été choisie en raison de sa capacité à produire des anticorps présentant une activité ADCC améliorée par rapport à des anticorps de même structure primaire produits par exemple dans CHO.

Dans un aspect particulier de l'invention, la lignée cellulaire stable exprimant un anticorps selon l'invention, et plus particulièrement choisie dans le groupe précédemment décrit, a intégré les deux vecteurs d'expression de la chaîne lourde et de la chaîne légère, tels que précédemment décrits.

Un aspect particulier de l'invention se rapporte au clone R565 déposé sous le numéro d'enregistrement I-3510 à la Collection Nationale de Cultures de Microorganismes (CNCM) .

La présente invention se rapporte également à toute lignée cellulaire produisant un anticorps monoclonal possédant une réactivité substantiellement similaire à celle de l'anticorps EMAB565 produit par la lignée R565 telle que décrite ci-dessus.

Un autre objet particulier de l'invention se rapporte à un anticorps monoclonal anti-idiotypique se liant à un anticorps dirigé contre le domaine C2 du FVIII humain et produit par le clone R565.

Un autre objet de l'invention se rapporte à un fragment d'ADN de séquence SEQ ID NO : 6 codant pour la chaîne lourde d'un anticorps selon l'invention. La séquence d'acide nucléique chimérique murine-humaine SEQ ID NO : 6 code pour chacune des chaînes lourdes de l'anticorps. Elle est obtenue par fusion de la séquence d'acide nucléique murine SEQ ID NO : 2 codant pour la région variable de chacune des chaînes lourdes de l'anticorps et de la séquence d'acide nucléique humaine SEQ ID NO : 3 codant pour la région constante de chacune des chaînes lourdes de l'anticorps.

Un autre objet particulier de l'invention se rapporte à un fragment d'ADN de séquence SEQ ID NO : 5 codant pour la chaîne légère d'un anticorps selon l'invention. La séquence d'acide nucléique chimérique murine-humaine SEQ ID NO : 5 code pour chacune des chaînes légères de l'anticorps. Elle est obtenue par fusion de la séquence d'acide nucléique murine SEQ ID NO : 1 codant pour la région variable de chacune des chaînes légères de l'anticorps et de la séquence d'acide nucléique humaine SEQ ID NO : 4 codant pour la région constante de chacune des chaînes légères de l'anticorps.

Un autre objet de l'invention est l'utilisation d'un anticorps anti-idiotypique, de manière avantageuse monoclonal, dirigé contre un anticorps inhibiteur du FVIII humain pour le recrutement, in vivo ou in vitro, par la région Fc dudit anticorps anti-idiotypique, de cellules immunitaires cytotoxiques. Une telle utilisation peut être mise en oeuvre avec tout anticorps anti-idiotypique cytotoxique dirigé contre tout domaine du FVIII. Ces anticorps cytotoxiques anti-idiotypiques vont activer les récepteurs FcγRIII, et notamment les récepteurs FcγRIIIA de cellules immunitaires cytotoxiques. De manière avantageuse, l'anticorps utilisé est un anticorps selon l'invention tel que décrit précédemment, et de manière particulièrement avantageuse, il s'agit de l'anticorps EMAB565.

Les anticorps de l'invention présentent la capacité d'activer par leur région Fc le récepteur FcγRIIIA. Ceci représente un intérêt considérable, car ce récepteur est exprimé à la surface de cellules appelées « cellules effectrices » : la liaison de la région Fc de l'anticorps à son récepteur porté par la cellule effectrice provoque l'activation du FcγRIIIA de la cellule effectrice et la destruction des cellules cibles. Les cellules effectrices sont par exemple des cellules NK (Natural Killer), des macrophages, des neutrophiles, les lymphocytes CD8, les lymphocytes Tγδ, les cellules NKT, les éosinophiles, les basophiles ou les mastocytes.

De manière avantageuse, une telle utilisation peut être effectuée pour la destruction, in vivo ou in vitro, de cellules précurseurs de plasmocytes sécréteurs d'anticorps inhibiteurs du FVIII, exprimant à leur surface un anticorps inhibiteur du FVIII. De préférence, ces cellules sont des cellules B, en particulier des cellules B mémoire. De manière avantageuse, cet anticorps inhibiteur est dirigé contre le domaine C2 du FVIII.

Les lymphocytes B expriment à leur surface un récepteur pour l'antigène (BCR pour « B-cell receptor ») qui est constitué d'une immunoglobuline membranaire associée à d'autres protéines. Chaque lymphocyte B ne synthétise qu'une seule variété d'immunoglobuline membranaire dont les régions variables sont identiques à celles des anticorps sécrétés. Par leur BCR, les lymphocytes B reconnaissent directement les antigènes. La liaison d'un antigène par le BCR, si elle est accompagnée d'autres signaux essentiels, peut déclencher la multiplication dudit lymphocyte B provoquant la formation d'un clone par multiplication de ce lymphocyte. Une partie des lymphocytes B issus des mitoses se différencie en plasmocytes sécréteurs d'anticorps circulants, l'autre partie formant des lymphocytes B mémoires, inactifs à l'issue de cette première réaction.

Les patients atteints d'une hémophilie A (en particulier l'hémophilie A sévère) n'ont pas ou très peu de FVIII endogène, c'est-à-dire que cette protéine est étrangère à leur organisme, et le FVIII administré peut déclencher une réaction immune lors de sa première administration ou une administration ultérieure. La liaison entre le FVIII administré et le BCR exprimé sur un lymphocyte B, sous condition qu'elle soit suffisamment affine, peut activer le lymphocyte B, résultant en la sécrétion d'anticorps anti-FVIII soluble par des plasmocytes et en la formation de lymphocytes B mémoires exprimant en surface un BCR spécifique au FVIII. Dans le cas d'un nouveau contact avec le FVIII, ces lymphocytes B mémoires se multiplient pour augmenter le nombre de lymphocytes B mémoires avec la même spécificité et se différencient en plasmocytes qui sécrètent des anticorps anti-FVIII avec une haute affinité.

La réaction engendrée par les lymphocytes B mémoires est plus intense que celle engendrée par les lymphocytes B naïfs, ces cellules mémoires étant plus nombreuses et ayant un BCR plus affin que les lymphocytes dont elles sont issues. Par ailleurs, la différenciation en plasmocytes est plus rapide à partir des cellules mémoires qu'à partir des cellules initiales de même spécificité.

L'immunoglobuline membranaire, due à sa spécificité par rapport à un certain antigène, est une caractéristique distinctive d'un lymphocyte B. Un lymphocyte B ayant un BCR anti-FVIII et donnant lieu à des plasmocytes sécréteurs d'anticorps inhibiteurs du FVIII, peut alors être ciblé par un anticorps se liant aux portions de l'immunoglobuline qui donnent la spécificité à l'anticorps anti-FVIII. Ainsi, l'anticorps selon l'invention reconnaît l'immunoglobuline membranaire, et, grâce au recrutement de cellules immunitaires cytotoxiques par sa région Fc, est responsable de la lyse des cellules exprimant le BCR à leur surface.

Plus particulièrement, une telle utilisation peut être effectuée pour la destruction, in vivo ou in vitro, de cellules B mémoires exprimant à leur surface un BCR ayant les régions variables des chaînes légères (VL) et les régions variables des chaînes lourdes (VH) de l'anticorps BO2C11 . Cette destruction a lieu via un mécanisme d'élimination cellulaire dépendante des anticorps, en particulier l'ADCC.

Un autre objet particulier de l'invention est l'utilisation de l'anticorps selon l'invention comme médicament. Avantageusement, un tel médicament est destiné à traiter les maladies dans lesquelles des anticorps inhibiteurs du FVIII sont produits.

Un autre objet de l'invention est l'utilisation d'un anticorps selon l'invention pour la fabrication d'un médicament.

A cet égard, un autre objet de l'invention est l'utilisation de l'anticorps selon l'invention pour la fabrication d'un médicament utilisé dans le traitement de l'hémophilie A. Aux fins de l'invention, l'expression « utilisé dans le traitement de l'hémophilie A » doit être comprise comme étant équivalente à l'expression « destiné au traitement de l'hémophilie A ».

De manière avantageuse, l'hémophilie A traitée est l'hémophilie A avec inhibiteurs.

L'anticorps anti-idiotypique selon l'invention peut être administré au patient en même temps que le FVIII, soit dans le même médicament, soit par deux administrations séparées mais concomitantes.

Enfin, un dernier objet de l'invention se rapporte à une composition pharmaceutique comprenant un anticorps selon l'invention et un ou plusieurs excipients et/ou véhicules pharmaceutiquement acceptables. L'excipient peut être toute solution, telle qu'une solution saline, physiologique, isotonique, tamponnée, etc., ainsi que toute suspension, gel, poudre, etc., compatible avec un usage pharmaceutique et connu de l'homme du métier. Les compositions selon l'invention peuvent en outre contenir un ou plusieurs agents ou véhicules choisis parmi les dispersants, solubilisants, stabilisants, surfactants, conservateurs, etc. D'autre part, les compositions selon l'invention peuvent comprendre d'autres agents ou principes actifs.

Par ailleurs, les compositions peuvent être administrées de différentes manières et sous différentes formes. L'administration peut être réalisée par toute voie classique pour ce type d'approche thérapeutique, comme notamment par voie systémique, en particulier par injection intraveineuse, intradermique, intra-tumorale, sous-cutanée, intra-péritonéale, intramusculaire, intra-artérielle, etc. On peut citer par exemple l'injection intra-tumorale ou l'injection dans une zone proche de la tumeur ou irriguant la tumeur.

Les doses peuvent varier en fonction du nombre d'administrations, de l'association à d'autres principes actifs, du stade d'évolution de la pathologie, etc.

Description des figures

Figure 1 : Lyse de BO2C11 induite par l'anticorps EMAB565 (noté 14C12 CH sur la figure) en présence de cellules NK humaines.

**Figure 2 :** Sécrétion d'IL2 par Jurkat CD16 en présence de BO2C11 et de l'anticorps EMAB565 (noté 14C12 CH sur la figure).

**Figure 3 :** Capping des BCR de BO2C11 induit par l'anticorps EMAB565 (noté 14C12 CH sur la figure).

**Figure 4 :** Apoptose de BO2C11 induite par l'anticorps EMAB565 (noté 14C12 CH sur la figure).

**Figure 5 :** Lyse de BO2C11 induite par l'anticorps EMAB565 (noté 14C12 CH sur la figure) en présence de complément.

## Exemples

### Exemple 1 : Construction des vecteurs d'expression de l'anticorps chimérique anti-Id FVIII EMAB565

A. Détermination de la séquence leader des régions variables de l'anticorps murin 14C12

L'ARN total de l'hybridome murin 14C12 produisant une immunoglobuline de type IgG2a,κ a été isolé. Après transcription inverse, les domaines variables des chaînes légères (Vκ) et lourdes (VH) de l'anticorps 14C12 ont été amplifiées par la technique de 5'RACE (Rapid Amplification of cDNA Ends) (kit GeneRacer, Invitrogen réf. L1500-01).

Brièvement, une première étape de transcription inverse a été tout d'abord réalisée en utilisant une amorce localisée dans la région 5' des régions constantes Cκ ou G1 murines. Une séquence poly-dC a été ensuite ajoutée en 3' des ADNc synthétisés avant de réaliser l'amplification des régions Vκ et VH à l'aide d'une amorce 5' reconnaissant la séquence poly-dC et d'une amorce 3', localisée dans les régions constantes Cκ ou G1 murines en 5' de l'amorce de transcription inverse. Les amorces utilisées pour ces deux étapes sont les suivantes :

1. amorces de transcription inverse

a. Amorce antisens spécifique Kappa murin (SEQ ID NO : 19)
5'- ACT GCC ATC AAT CTT CCA CTT GAC -3'
b. Amorce antisens spécifique G2a murin (SEQ ID NO : 20)
5'- CTG AGG GTG TAG AGG TCA GAC TG -3'

2. amorces de PCR 5'RACE

a. Amorce antisens spécifique Kappa murin (SEQ ID NO : 21)
5'- TTGTTCAAGAAGCACACGACTGAGGCAC -3'
b. Amorce antisens spécifique G2a murin (SEQ ID NO : 22)
5'- GAGTTCCAGGTCAAGGTCACTGGCTCAG -3'

Les produits de PCR VH et Vκ ainsi obtenus ont été clonés dans le vecteur pCR4Blunt-TOPO (Zero blunt TOPO PCR cloning kit, Invitrogen, réf. K2875-20) puis séquencés. La séquence nucléotidique de la région Vκ de l'anticorps murin 14C12 est indiquée sous la séquence SEQ ID NO : 1 et la séquence peptidique déduite est la séquence SEQ ID NO : 9. Le gène Vκ appartient au sous-groupe Vκ23 [Almagro JC et al Immunogenetics (1998), 47 : 355-363]. Les séquences des CDR1, CDR2 et CDR3 de la région Vκ de l'anticorps murin 14C12, définies selon la numérotation de Kabat [Kabat et al., "Sequences of Proteins of Immunological Interest", NIH Publication, 91-3242 (1991)], sont indiquées sous les séquences suivantes : SEQ ID NO : 13, SEQ ID NO : 14 et SEQ ID NO : 15, respectivement. Les séquences des CDR1-IMGT, CDR2-IMGT et CDR3-IMGT de la région Vκ de l'anticorps murin 14C12, définies selon l'analyse IMGT (international ImMunoGeneTics database) [Lefranc, M.-P. et al., Dev. Comp. Immunol., 27, 55-77 (2003)] sont indiquées sous les séquences suivantes : SEQ ID NO : 27, SEQ ID NO : 28 et SEQ ID NO : 29, respectivement. Cette définition, différente de celle de Kabat fondée sur la seule analyse de variabilité des séquences, prend en compte et combine la caractérisation des boucles hypervariables [Chothia C. and Lesk A.M. J. Mol. Biol. 196 : 901-17 (1987)] et l'analyse structurale des anticorps par cristallographie.

La séquence nucléotidique de la région VH de 14C12 est la séquence SEQ ID NO : 2 et la séquence peptidique déduite est la séquence SEQ ID NO : 10. Le gène VH appartient au sous-groupe VH1 [Honjo T. and Matsuda F. in « Immunoglobulin genes ». Honjo T. and Alt F.W. eds, Academic Press, London (1996), pp145-171] . Les séquences des CDR1, CDR2 et CDR3 de la région VH de l'anticorps murin 14C12, définies selon la numérotation de Kabat [Kabat et al., "Séquences of Proteins of Immunological Interest", NIH Publication, 91-3242 (1991)], sont indiquées sous les séquences suivantes : SEQ ID NO : 16, SEQ ID NO : 17 et SEQ ID NO : 18, respectivement. Les séquences des CDR1-IMGT, CDR2-IMGT et CDR3-IMGT de la région VH de l'anticorps murin 14C12, définies selon l'analyse IMGT (international ImMunoGeneTics database) [Lefranc, M.-P. et al., Dev. Comp. Immunol., 27, 55-77 (2003)] sont indiquées sous les séquences suivantes : SEQ ID NO : 30, SEQ ID NO : 31 et SEQ ID NO : 32, respectivement. Cette définition, différente de celle de Kabat fondée sur la seule analyse de variabilité des séquences, prend en compte et combine la caractérisation des boucles hypervariables [Chothia C. and Lesk A.M. J. Mol. Biol. 196 : 901-17 (1987)] et l'analyse structurale des anticorps par cristallographie.

B. Construction des vecteurs d'expression chaîne lourde et chaîne légère de l'anticorps chimérique EMAB565

1. Vecteur chaîne légère Kappa

La séquence Vκ clonée dans le vecteur de séquençage pCR4Blunt-TOPO a été amplifiée à l'aide des amorces de clonage suivantes :

a) amorce sens Vκ (SEQ ID NO : 23) 5'- GTATACTAGT**GCCGCCACC**ATGGTTTCACACCTCAGAT -3'
La séquence soulignée correspond au site de restriction Spe I, la séquence en gras correspond à une séquence de Kozak consensus, l'ATG initiateur est en italique.
b) amorce antisens Vκ (SEQ ID NO : 24) 5'- **TGAAGACACTTGGTGCAGCCACAGTCCG**TTTTATTTCCAACTTGGTC -3'
Cette amorce réalise la jonction des séquences Vκ murine (en italique) et région constante (Cκ) humaine (en gras). La séquence soulignée correspond au site de restriction Dra III.

Le produit de PCR Vκ ainsi obtenu contient la séquence codant le peptide signal naturel de l'anticorps murin 14C12. Cette PCR Vκ a été ensuite clonée entre les sites Spe I et Dra III du vecteur de chimérisation chaîne légère en 5' de la région constante Cκ humaine, dont la séquence nucléique est la séquence SEQ ID NO : 4 et la séquence peptidique déduite est la séquence SEQ ID NO 12. La séquence Cκ humaine de ce vecteur de chimérisation a été préalablement modifiée par mutagénèse silencieuse afin de créer un site de restriction Dra III pour permettre le clonage de séquences Vκ murines. Ce vecteur de chimérisation contient un promoteur bien connu de l'homme du métier, comme CMV ou RSV et une séquence de polyadénylation HGH (human Growth Hormone) ainsi que le gène de sélection dhfr (dihydrofolate reductase).

Pour l'expression de l'anticorps EMAB565, le promoteur du vecteur de chimérisation a été ensuite remplacé par le promoteur EF-1 alpha humain.

La séquence de la chaîne légère de l'anticorps chimérique EMAB565 codée par ce vecteur est présentée en SEQ ID NO : 5 pour la séquence nucléotidique et correspond à la séquence peptidique déduite SEQ ID NO : 7.

2. Vecteur chaîne lourde

Une démarche similaire a été appliquée pour la chimérisation de la chaîne lourde de l'anticorps EMAB565. La séquence VH clonée dans le vecteur pCR4Blunt-TOPO a été tout d'abord amplifiée à l'aide des amorces de clonage suivantes :

a) amorce sens VH (SEQ ID NO : 25) 5'- CTATTACTAGT**GCCGCCACC**ATGGAATGGAGTTGGATATTT -3'
La séquence soulignée correspond au site de restriction Spe I, la séquence en gras correspond à une séquence de Kozak consensus, l'ATG initiateur est en italique.

b) amorce antisens VH (SEQ ID NO : 26) 5'- **GACCGATGGGCCCTTGGTGGAGGC**TGAGGAGACGGTGACCGTG -3'
Cette amorce réalise la jonction des séquences VH murine (en italique) et région constante G1 humaine (en gras). La séquence soulignée correspond au site de restriction Apa I.

Le fragment VH amplifié contient la séquence codant le peptide signal naturel de l'anticorps murin 14C12. Cette PCR VH a été ensuite clonée entre les sites Spe I et Apa I du vecteur de chimérisation chaîne lourde en 5' de la région constante γ1 humaine dont la séquence nucléique est la séquence SEQ ID NO : 3 et la séquence peptidique déduite est la séquence SEQ ID NO : 11. Ce vecteur de chimérisation contient un promoteur bien connu de l'homme du métier, comme CMV ou RSV et une séquence de polyadénylation bGH (bovine Growth Hormone) ainsi que le gène de sélection neo.

Pour l'expression de l'anticorps EMAB565, le promoteur du vecteur de chimérisation a été ensuite remplacé par le promoteur EF-1 alpha humain.

La séquence de la chaîne lourde de l'anticorps chimérique EMAB565 codée par ce vecteur est présenté en SEQ ID NO : 6 pour la séquence nucléotidique et en séquence SEQ ID NO : 8 pour la séquence peptidique déduite.

**Exemple 2 : Création d'une lignée cellulaire dérivée de la lignée YB2/0 productrice de l'anticorps chimérique anti-inhibiteur du FVIII EMAB565**

La lignée de rat YB2/0 (ATCC # CRL-1662) a été cultivée en milieu EMS (Invitrogen, réf. 041-95181M) contenant 5% de sérum de veau foetal (SVF) (JRH Biosciences, réf. 12107). Pour la transfection, 5 millions de cellules ont été électroporés (électroporateur Biorad, modèle 1652077) en milieu Optimix (Equibio, réf. EKITE 1) avec 25 μg de vecteur chaîne légère linéarisé par Aat II, et 27 μg de vecteur chaîne lourde linéarisé par Sca I. Les conditions d'électroporation appliquées étaient de 230 volts et 960 microfarads pour une cuvette de 0,5 ml. Chaque cuvette d'électroporation a été ensuite répartie sur 5 plaques P96 avec une densité de 5000 cellules/puits.

La mise en milieu sélectif RPMI (Invitrogen, ref 21875-034) contenant 5% de sérum dialysé (Invitrogen, réf. 10603-017), 500 μg/ml de généticine G418 (Invitrogen, réf. 10131-027) et 25 nM de methotrexate (Sigma, réf. M8407), a été réalisée 3 jours après la transfection.

Les surnageants des puits de transfection résistants ont été criblés pour la présence d'immunoglobuline (Ig) chimérique par dosage ELISA spécifique des séquences Ig humaines.

Les 10 transfectants produisant le plus d'anticorps ont été amplifiés en plaques P24 et leur surnageant redosé par ELISA afin d'estimer leur productivité et de sélectionner les 3 meilleurs producteurs pour le clonage par dilution limite (40 cellules / plaque).

A l'issue du clonage, le clone R565, dénommé ci-après « R565 », a été sélectionné pour la production de l'anticorps chimérique EMAB565 et adapté progressivement au milieu de production CD Hybridoma (Invitrogen, réf. 11279-023).

La production de l'anticorps chimérique EMAB565 a été réalisée par expansion de la culture adaptée en milieu CD Hybridoma, obtenue par dilution à $3x10^5$ cellules/ml en flacons de 75 $cm^2$ et 175 $cm^2$ puis par dilution à $4,5x10^5$ cellules/ml en flacon de type roller. Après avoir atteint le volume maximal (1 1), la culture a été poursuivie jusqu'à ce que la viabilité cellulaire ne soit plus que de 20%. Après production, l'anticorps chimérique EMAB565 a été purifié par chromatographie d'affinité sur protéine A (pureté estimée par HPLC < 95 %) et contrôlé par électrophorèse en gel de polyacrylamide.

L'analyse glycannique de la composition d'anticorps produite (EMAB565) a été effectuée par HPCE-LIF et montre une teneur en fucose d'environ 7%, une teneur en galactose d'environ 52% et un ratio fuc/Gal égal à 0,133.

Exemple 3 : Lyse de B02C11 induite par l'anticorps EMAB565 en présence de cellules NK humaines

Technique ADCC

Les cellules NK sont isolées à partir des PBMC (Peripheral Blood Mononuclear Cells) en utilisant la technique de séparation sur billes magnétiques (MACS) de chez Myltenyi. Les cellules NK sont lavées et resuspendues en IMDM (Iscove's modified Dubelcco's Medium) +5% SVF ($45x10^5$ cellules/ml). Les cellules effectrices et les cellules cibles sont utilisées dans un ratio 15/1. Les cellules cibles BO2C11 sont ajustées à $3x10^5$ cellules/ml en IMDM 5% SVF. Les anticorps sont dilués en IMDM 0,5% SVF (concentration finale 500 ; 50 ; 5 ; 0,5 ; 0,005 et 0,005 ng/ml).

Le mélange réactionnel est composé de 50μl d'anticorps, 50μl de cellules effectrices, 50μl de cellules cibles et de 50μl de milieu IMDM en plaque de microtitration P96. Deux témoins négatifs sont établis :

- Lyse sans NK : les cellules effectrices NK sont remplacées par de l'IMDM + 5% SVF.
- Lyse sans anticorps (Ac) : les anticorps sont remplacées par de l'IMDM + 5% SVF.

Après 16h d'incubation à 37°C sous atmosphère enrichie de 5% $CO_2$, les plaques sont centrifugées et les taux de LDH intracellulaire libérée dans le surnageant évalués par un réactif spécifique (Cytotoxicity Detection Kit 1 644 793).

Le pourcentage de lyse est estimé en utilisant une gamme de calibration obtenue avec différentes dilutions de cellules cibles lysées au Triton X100 (2%) correspondant à 100, 50, 25 et 0% de lyse respectivement.

Les résultats sont calculés selon la formule suivante :

% de lyse = (%lyse avec Anticorps et NK) - (%lyse sans Anticorps) - (% de lyse sans NK).

Les anticorps anti-idiotype des inhibiteurs du FVIII 14C12 murin et 14C12 chimérique EMABling sont étudiés pour leur capacité à lyser les cellules BO2C11 en présence de cellules NK.

La Figure 1 montre que l'anticorps 14C12 murin n'induit pas de lyse des cellules BO2C11 alors que l'anticorps chimérique (EMABling Technology) induit une lyse dose dépendante.

**Exemple 4 : Sécrétion d'IL-2 par Jurkat CD16 en présence de BO2C11 et de l'anticorps EMAB565**

Ce test estime la capacité des anticorps à se fixer sur le récepteur CD16 (Fc gamma RIIIa) exprimé sur les cellules Jurkat CD16 et à induire la sécrétion d'IL2. Pour cela, on mélange en plaque de 96 puits :

- 50μl d'une solution d'anticorps (concentration finale à 25, 2,5, 0,25, 0,025, 0,012 μg/ml en IMDM 5% SVF),
- 50μl de PMA (dilution à 40ng/ml en IMDM 5% SVF), 50μl de cellules BO2C11 diluées à $6x10^5$/ml en IMDM 5% SVF, et 50μl de cellules Jurkat CD16 ($20x10^6$/ml en IMDM 5% SVF). Après incubation 1 nuit à 37°C, les plaques sont centrifugées, et l'IL2 contenue dans les surnageants évaluée par le kit commercial (Quantikine de chez R/D). La lecture de la DO se fait à 450nm.

Les résultats sont exprimés en taux d'IL-2 en fonction de la concentration d'anticorps.

L'anticorps anti-idiotype des inhibiteurs du FVIII EMAB565 est étudié pour sa capacité à induire la sécrétion d'IL2 de cellules Jurkat transfectées par le CD16 en présence de cellules BO2C11.

La Figure 2 montre que l'anticorps EMAB565 induit la sécrétion d'IL-2.

**Exemple 5 : Capping des BCR de BO2C11 induit par l'anticorps EMAB565**

Les cellules BO2C11 (1,2x$10^6$ cellules/ml) sont lavées et conservées en IMDM contenant 5% SVF pH 7.4. Les cellules sont incubées 1h, 4h et 16h à 4°C et 37°C avec l'anticorps EMAB565 préalablement marqué (ALEXA) à la concentration de 10 $\mu$g/ml. Le capping, c'est-à-dire le coiffage des récepteurs par l'anticorps, est visualisé au microscope LEICA à fluorescence (objectif x63 sous huile à immersion).

Différents temps d'incubation ont été étudiés et montrent que l'intensité du capping est précoce (30 minutes) et s'intensifie au cours du temps. A titre d'exemple, la figure 4 montre qu'à 16h, les anticorps fixés sur BO2C11 sont regroupés sur des pôles et non répartis uniformément sur la surface de la membrane. Ceci indique que la fixation de l'anticorps 14C12 CH sur BO2C11 induit un réarrangement des récepteurs membranaires et potentiellement l'induction d'un signal de transduction.

**Exemple 6 : Apoptose de BO2C11 induite par l'anticorps EMAB565**

Les cellules BO2C11 (2,5 x $10^5$) sont incubées avec l'anticorps EMAB565 (1$\mu$g/ml) avec ou sans crosslinker (F(ab2)' de chèvre anti-Fc$\gamma$ d'IgG humaines à 10 $\mu$g/ml) dans 1 ml de RMPI 10% SVF, dans des plaques P24 pendant 24h à 37°C. Les cellules sont ensuite centrifugées, lavées deux fois en PBS, reprises dans le tampon fourni par le kit et incubées avec l'AnnexineV-FITC et l'iodure de propidium (IP) selon les recommandations de BD Biosciences. Les cellules sont analysées au cytomètre en flux, le pourcentage de cellules apoptotiques correspond aux cellules marquées par l'annexine V (annexine V et annexine V +IP).

La Figure 4 montre que l'induction d'apoptose en présence de l'anticorps EMAB565 (technologie EMABling) est inférieure à 3% en absence de cross linker et seulement de 6,5% en présence de cross-linker (figure 4).

**Exemple 7 : Lyse de B02C11 induite par l'anticorps EMAB565 en présence de complément**

Les cellules BO2C11 sont lavées et incubées pendant 1h à 37°C avec différentes concentrations de l'anticorps EMAB565 (concentration finale de 0 à 2,5$\mu$g/ml) en présence de source de complément (Young rabbit complement de Cedarlane) dilué au 1:10 en milieu IMDM + SVF 5%. Les cellules sont ensuite centrifugées 2 fois à 1200 tours/min (270 g) pendant 1 minute et les surnageants sont prélevés. La quantité de LDH intracellulaire libérée dans le surnageant correspondant à la lyse cellulaire est mesurée par un réactif spécifique (Cytotoxicity Detection Kit 1 644 793).

Le pourcentage de lyse est estimé en utilisant une gamme de calibration obtenue avec différentes dilutions de cellules cibles lysées au Triton X-100 (2%) correspondant à 100, 50, 25 et 0% de lyse respectivement. Les contrôles incluent le relargage spontané (cellules cibles seules).

Les résultats sont calculés selon la formule suivante :

% de lyse = (%lyse avec Anticorps et complément) - (% de lyse sans complément).

Sur la Figure 5 on observe que l'activité CDC (Complement Dependant Cytotoxicity) induite par l'anticorps EMAB565 (technologie EMABling) est au maximum de 5% pour les plus fortes concentrations d'anticorps utilisées.

## Revendications

1. Anticorps monoclonal anti-idiotypique dirigé contre un anticorps inhibiteur du facteur VIII (FVIII) humain, ledit anticorps inhibiteur étant dirigé contre la région C2 du FVIII humain, **caractérisé en ce que** la région variable de chacune de ses chaînes légères est codée par une séquence d'acides nucléiques possédant au moins 70% d'identité avec la séquence d'acides nucléiques murine SEQ ID NO : 1, la région variable de chacune de ses chaînes lourdes est codée par une séquence d'acides nucléiques possédant au moins 70% d'identité avec la séquence d'acides nucléiques murine SEQ ID NO : 2, et les régions constantes de ses chaînes légères et de ses chaînes lourdes sont des régions constantes humaines respectivement codées par la séquence d'acide nucléique SEQ ID NO : 4 et par la séquence d'acide nucléique humaine SEQ ID NO : 3.

2. Anticorps monoclonal anti-idiotypique selon la revendication 1, **caractérisé en ce que** la région variable de chacune de ses chaînes légères est codée par la séquence d'acides nucléiques possédant au moins 80% d'identité avec la séquence d'acides nucléiques murine SEQ ID NO : 1, la région variable de chacune de ses chaînes lourdes est codée par la séquence d'acides nucléiques possédant au moins 80% d'identité avec la séquence d'acides nucléiques murine SEQ ID NO : 2.

3. Anticorps monoclonal anti-idiotypique selon la revendication 1, **caractérisé en ce que** la région variable de chacune de ses chaînes légères est codée par la séquence d'acides nucléiques possédant au moins 95% d'identité avec la séquence d'acides nucléiques murine SEQ ID NO : 1, la région variable de chacune de ses chaînes lourdes est codée par la séquence d'acides nucléiques possédant au moins 95% d'identité avec la séquence d'acides nucléiques murine SEQ ID NO : 2.

4. Anticorps monoclonal anti-idiotypique selon la revendication 1, **caractérisé en ce que** la région variable de chacune de ses chaînes légères est codée par la séquence d'acides nucléiques possédant au moins 99% d'identité avec la séquence d'acides nucléiques murine SEQ ID NO : 1, la région variable de chacune de ses chaînes lourdes est codée par la séquence d'acides nucléiques possédant au moins 99% d'identité avec la séquence d'acides nucléiques murine SEQ ID NO : 2.

5. Anticorps monoclonal anti-idiotypique selon la revendication 1, **caractérisé en ce que** la région variable de chacune de ses chaînes légères est codée par la séquence d'acides nucléiques murine SEQ ID NO : 1, la région variable de chacune de ses chaînes lourdes est codée par la séquence d'acides nucléiques murine SEQ ID NO : 2.

6. Anticorps monoclonal anti-idiotypique selon l'une quelconque des revendications précédentes, **caractérisé en ce qu'**il est produit par une cellule sous forme de composition d'anticorps, dont le ratio taux de fucose/taux de galactose des structures glycanniques présentes sur le site de glycosylation de la région Fc, est inférieur ou égal à 0,6.

7. Anticorps monoclonal anti-idiotypique selon l'une quelconque des revendications précédentes, **caractérisé en ce que** ledit anticorps inhibiteur du FVIII est l'anticorps BO2C11.

8. Anticorps monoclonal anti-idiotypique selon l'une quelconque des revendications précédentes, **caractérisé en ce que** chacune de ses chaînes légères est codée par une séquence en acides nucléiques possédant au moins 70% d'identité avec la séquence d'acide nucléique chimérique murine humaine SEQ ID NO : 5 et **en ce que** chacune de ses chaînes lourdes est codée par une séquence possédant au moins 70% d'identité avec la séquence d'acide nucléique chimérique murine-humaine SEQ ID NO : 6.

9. Anticorps monoclonal anti-idiotypique selon l'une quelconque des revendications précédentes, **caractérisé en ce que** chacune de ses chaînes légères est codée par la séquence d'acides nucléiques chimérique murine-humaine SEQ ID NO : 5 et **en ce que** chacune de ses chaînes lourdes est codée par la séquence d'acide nucléique chimérique murine-humaine SEQ ID NO : 6.

10. Anticorps monoclonal anti-idiotypique selon l'une quelconque des revendications précédentes, **caractérisé en ce que** chacune de ses chaînes légères possède une séquence peptidique ayant au moins 70% d'identité avec la séquence SEQ ID NO : 7 et **en ce que** chacune de ses chaînes lourdes possède une séquence peptidique ayant au moins 70% d'identité avec la séquence SEQ ID NO : 8.

11. Anticorps monoclonal anti-idiotypique selon l'une quelconque des revendications précédentes, **caractérisé en ce que** la séquence peptidique de chacune de ses chaînes légères est la séquence peptidique SEQ ID NO : 7 et **en ce que** la séquence peptidique de chacune de ses chaînes lourdes est la séquence peptidique SEQ ID NO : 8.

12. Anticorps monoclonal anti-idiotypique selon l'une des revendications précédentes, **caractérisé en ce que** qu'il est produit dans l'hybridome de rat YB2/0 (cellule YB2/3HL.P2.G11.16Ag.20, déposée à l'American Type Culture Collection sous le numéro ATCC CRL-1662).

13. Anticorps monoclonal anti-idiotypique selon l'une des revendications précédentes, **caractérisé en ce qu'**il est susceptible d'être produit par le clone R565 déposé sous le numéro d'enregistrement I-3510 à la Collection Nationale de Cultures de Microorganismes.

14. Anticorps monoclonal anti-idiotypique selon l'une quelconque des revendications précédentes, **caractérisé en ce qu'**il s'agit de l'anticorps EMAB565 produit par le clone R565 déposé sous le numéro d'enregistrement I-3510 à la Collection Nationale de Cultures de Microorganismes.

15. Fragment d'ADN de séquence SEQ ID NO : 6 codant pour la chaîne lourde d'un anticorps selon l'une des revendications précédentes.

16. Fragment d'ADN de séquence SEQ ID NO : 5 codant pour la chaîne légère d'un anticorps selon l'une des revendications précédentes.

**17.** Utilisation d'un anticorps monoclonal antiidiotypique selon l'une des revendications précédentes dirigé contre un anticorps inhibiteur du FVIII humain pour le recrutement, in vitro, par la région Fc dudit anticorps anti-idiotypique, de cellules immunitaires cytotoxiques.

**18.** Utilisation d'un anticorps monoclonal anti-idiotypique selon l'une des revendications précédentes, pour la destruction in vitro de cellules B, en particulier les cellules B mémoire, exprimant à leur surface un anticorps inhibiteur du FVIII.

**19.** Utilisation d'un anticorps selon l'une des revendications précédentes, pour la fabrication d'un médicament utilisé dans le traitement de l'hémophilie A.

**20.** Composition pharmaceutique comprenant un anticorps monoclonal anti-idiotypique selon des revendications précédentes et un ou plusieurs excipients et/ou véhicules pharmaceutiquement acceptables.

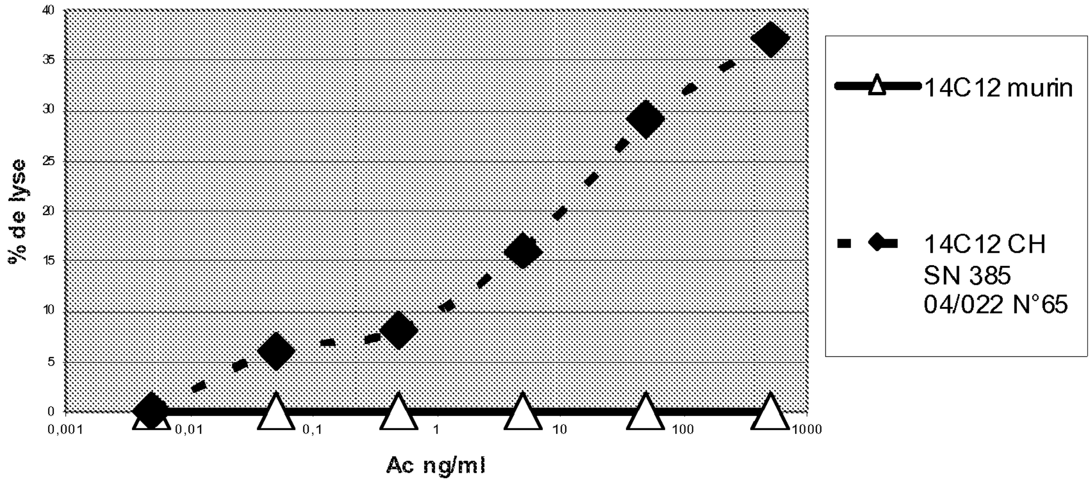
40
35
30
25
20
15
10
5
0
% de lyse
0,001
0,01
0,1
1
10
100
1000
Ac ng/ml
14C12 murin
14C12 CH
SN 385
04/022 N°65

Fig. 1

Fig.2

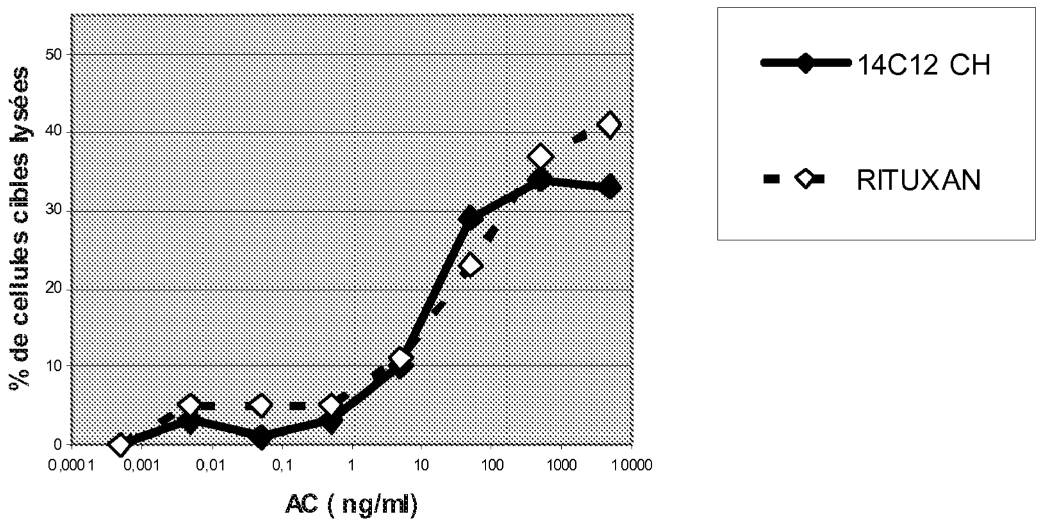
% de cellules cibles lysées
50
40
30
20
10
0
0,0001
0,001
0,01
0,1
1
10
100
1000
10000
AC ( ng/ml)
14C12 CH
RITUXAN

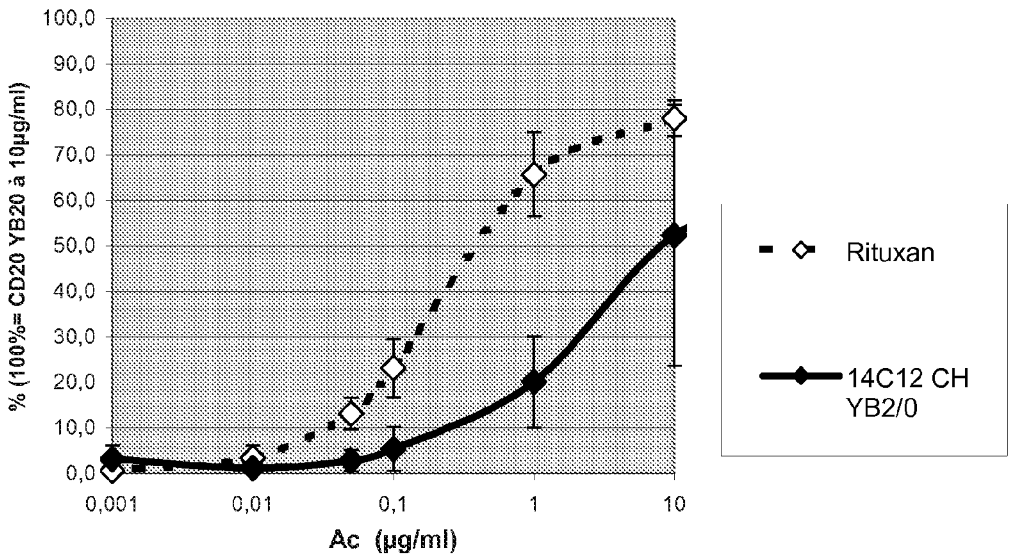
100,0
90,0
80,0
70,0
60,0
50,0
40,0
30,0
20,0
10,0
0,0
% (100%= CD20 YB20 à 10µg/ml)
0,001
0,01
0,1
1
10
Ac (µg/ml)
Rituxan
14C12 CH YB2/0

Fig.3

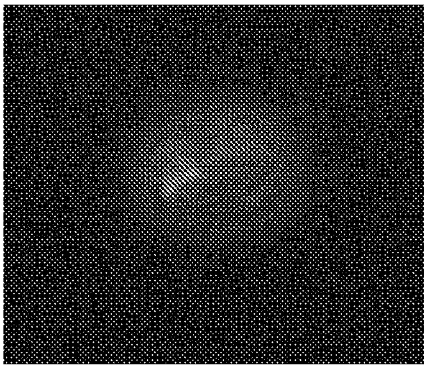

T0

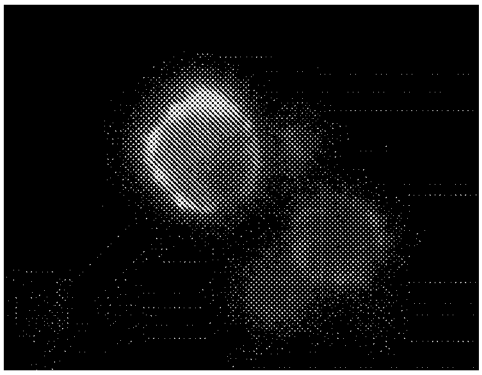

T16

Fig.4

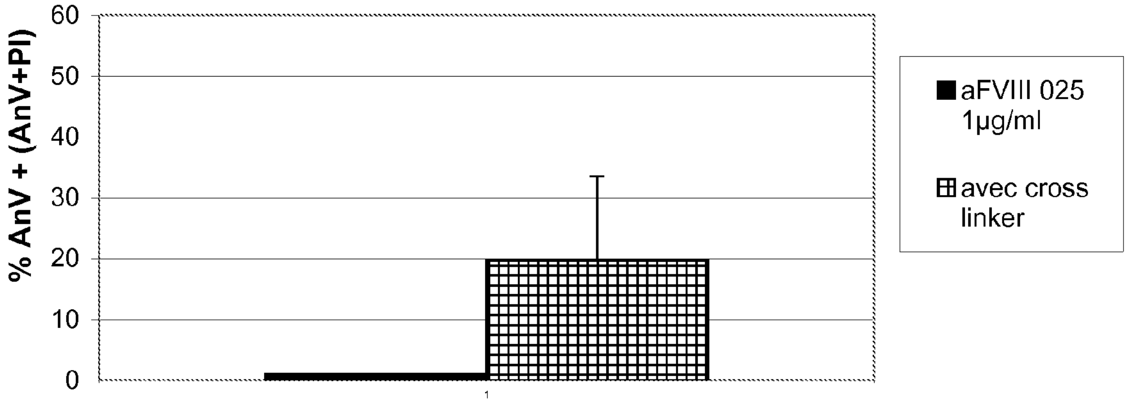
% AnV + (AnV+PI)
60
50
40
30
20
10
0
1
aFVIII 025 1µg/ml
avec cross linker

Fig.5

Europäisches Patentamt
European Patent Office
Office européen des brevets

# RAPPORT DE RECHERCHE EUROPEENNE

Numéro de la demande
EP 11 16 7006

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | WO 2004/014955 A (D. COLLEN RESEARCH FOUNDATION VZW; GILLES, JEAN-GUY, G; SAINT-REMY, JE) 19 février 2004 (2004-02-19) * pages 7-10 et 17-20; exemples 9 et 10 * | 1-20 | INV. A61K39/395 A61P7/04 C07K16/42 |
| Y | FR 2 861 080 A (LABORATOIRE FRANCAIS DU FRACTIONNEMENT ET DES BIOTECHNOLOGIES) 22 avril 2005 (2005-04-22) * le document en entier * | 1-20 | |
| Y | WO 2004/087757 A (LABORATOIRE FRANCAIS DU FRACTIONNEMENT ET DES BIOTECHNOLOGIES; INSTITU) 14 octobre 2004 (2004-10-14) * page 35 - page 36; séquence 5 * | 1-20 | |
| Y | US 2005/232931 A1 (MA JING ET AL) 20 octobre 2005 (2005-10-20) * exemple 3; séquence 13 * | 1-20 | |
| A | LITTLE M ET AL: "Of mice and men: hybridoma and recombinant antibodies", IMMUNOLOGY TODAY, ELSEVIER PUBLICATIONS, CAMBRIDGE, GB, vol. 21, no. 8, 1 août 2000 (2000-08-01), pages 364-370, XP004215163, ISSN: 0167-5699 * le document en entier * | 1-20 | DOMAINES TECHNIQUES RECHERCHES (IPC): C07K |

Le présent rapport a été établi pour toutes les revendications

1

| Lieu de la recherche | Date d'achèvement de la recherche | Examinateur |
|---|---|---|
| Munich | 19 octobre 2011 | Renggli, John |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 11 16 7006

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-10-2011

| Document brevet cité au rapport de recherche | Date de publication | Membre(s) de la famille de brevet(s) | Date de publication |
|---|---|---|---|
| WO 2004014955 A | 19-02-2004 | AT 508140 T | 15-05-2011 |
| | | AU 2003260335 A1 | 25-02-2004 |
| | | CA 2494698 A1 | 19-02-2004 |
| | | DK 1581560 T3 | 22-08-2011 |
| | | EP 1388544 A1 | 11-02-2004 |
| | | EP 1581560 A1 | 05-10-2005 |
| | | ES 2366280 T3 | 18-10-2011 |
| | | JP 2006515565 A | 01-06-2006 |
| | | PT 1581560 E | 17-08-2011 |
| | | US 2006239998 A1 | 26-10-2006 |
| FR 2861080 A | 22-04-2005 | AU 2004283924 A1 | 06-05-2005 |
| | | BR PI0415565 A | 02-01-2007 |
| | | CA 2542881 A1 | 06-05-2005 |
| | | EP 1675873 A1 | 05-07-2006 |
| | | WO 2005040221 A1 | 06-05-2005 |
| | | JP 2007533299 A | 22-11-2007 |
| | | US 2007015239 A1 | 18-01-2007 |
| WO 2004087757 A | 14-10-2004 | AU 2004226167 A1 | 14-10-2004 |
| | | CA 2520820 A1 | 14-10-2004 |
| | | EP 1613654 A2 | 11-01-2006 |
| | | JP 2007527696 A | 04-10-2007 |
| | | US 2007135621 A1 | 14-06-2007 |
| US 2005232931 A1 | 20-10-2005 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004014955 A
- EP 173494 A
- EP 125023 A
- WO 0104269 A
- FR 0312229


### Littérature non-brevet citée dans la description


- **WOOD et al.** *Nature,* 1984, vol. 312, 330-337
- **GILLES JG et al.** *Blood,* 1993, vol. 82, 2452-2461
- **JARVIS et al.** *Thromb Haemost.,* Février 1996, vol. 75 (2), 318-25
- **SAINT-RÉMY JM et al.** *Vox Sang,* 1999, vol. 77 (1), 21-24
- **MORRISON et al.** *Proc. Natl. Acad. Sci. U.S.A.,* 1984, vol. 81, 6851-55
- **NEUBERGER, M.S. et al.** *Nature,* 1985, vol. 312 (5995), 604-8
- **JACQUEMIN et al.** *Blood,* 1998, vol. 92, 496-506
- **JACQUEMIN MG et al.** *Blood,* 1998, vol. 92, 496-506
- **SPIEGEL PC et al.** *Blood,* 2001, vol. 98, 13-19
- **ALMAGRO JC et al.** *Immunogenetics,* 1998, vol. 47, 355-363
- **KABAT et al.** Sequences of Proteins of Immunological Interest. NIH Publication, 1991
- **LEFRANC, M.-P. et al.** *Dev. Comp. Immunol.,* 2003, vol. 27, 55-77
- **CHOTHIA C. ; LESK A.M.** *J. Mol. Biol.,* 1987, vol. 196, 901-17
- **HONJO T. ; MATSUDA F.** Immunoglobulin genes. Academic Press, 1996, 145-171
- **KABAT et al.** Séquences of Proteins of Immunological Interest. NIH Publication, 1991, 91-3242